# EUROPEAN PATENT SPECIFICATION

(11) **EP 2 486 123 B1**
(45) Date of publication and mention of the grant of the patent: **09.03.2016**
(21) Application number: 10768597.6
(22) Date of filing: 05.10.2010
(51) Int. Cl.: C12N 1/20, C12P 13/12

(54) **A METHOD FOR PRODUCING AN L-CYSTEINE, L-CYSTINE, A DERIVATIVE OR PRECURSOR THEREOF OR A MIXTURE THEREOF USING A BACTERIUM OF Enterobacteriaceae FAMILY**
VERFAHREN ZUR HERSTELLUNG VON L-CYSTEIN, L-CYSTIN, EINES DERIVATS ODER VORLÄUFERS DAVON ODER EINER MISCHUNG DAVON MIT EINEM BAKTERIUM DER ENTEROBACTERIACEAE-FAMILIE
PROCÉDÉ DE PRODUCTION DE L-CYSTÉINE, L-CYSTINE, D'UN DE LEURS DÉRIVÉS OU PRÉCURSEURS OU D'UN MÉLANGE DE CEUX-CI À L'AIDE D'UNE BACTÉRIE DE LA FAMILLE DES ENTEROBACTERIACEAE

(30) Priority: 05.10.2009 RU 2009136544
(43) Date of publication of application: 15.08.2012
(73) Proprietor: Ajinomoto Co., Inc., Tokyo 104-8315 (JP)
(72) Inventor: ZIYATDINOV, Mikhail Kharisovich, Moscow 115561 (RU); SAMSONOV, Viktor Vasilievich, Moscow 119313 (RU); GUSYATINER, Mikhail Markovich, Moscow 117648 (RU)
(74) Representative: Strehl Schübel-Hopf & Partner
(86) International application number: PCT/JP2010/067816
(87) International publication number: WO 2011/043485

(56) References cited:
- WO-A1-2007/012078
- WO-A1-2007/077041
- WO-A1-2008/127240
- WO-A2-03/006666

### SEQUENCE LISTING

<110> Ajinomoto Co., Inc.
<120> A METHOD FOR PRODUCING L-CYSTEINE, L-CYSTINE, A DERIVATIVE OR PRECURSOR THEREOF OR A MIXTURE THEREOF USING A BACTERIUM OF *Enterobacteriaceae* FAMILY
<130> D122-10164
<150> RU2009136544
   <151> 2009-10-05
<160> 66
<170> PatentIn version 3.5
<210> 1
   <211> 1416
   <212> DNA
   <213> Pantoea ananatis
<400> 1
   gtggattatt tgcctctttt tgccgatctc gcaggtcgac ccgtactggt cgtcggcggc      60
   ggagatatcg cggcgcgcaa gattgagctg ctgcgtcggg ccggggcgcg cattcaaatc     120
   gcctcacgcg aactctgccc cgagttacag gctttgctgg atgaacagca gcttgaatgg     180
   ctggccacgt cctttgaacc cgctcagctc gacaaggtct ttctggtcat tgccgctacc     240
   gatgacaatg cgctgaatgc gcaggtctat gacgaagcga atgcccgcca caagctggta     300
   aacgtggtag acgatcagcc taaatgcagc tttattttcc cctctattgt tgatcgatcg     360
   ccactggtcg tggcgatctc ctccagtggc accgcgccgg tgctggcccg catgctgcgc     420
   gagaaactcg aaacgctgct gccatcccat ctgggccaaa tggccgagct ggcgggtcag     480
   tggcgtgaca aagtcaaagc tcgcttcagc cgtatgtccg atcgccgtcg ttactgggaa     540
   agaatattta atggccgttt tgccagtcag atggcgacgg gcgacgttac ggccgctaaa     600
   cagacgctgg atagcgaact gggcgatcag ccgccccgac agggcgaaat tattctggtt     660
   ggcgcggggc ctggcgacag cggcctgtta accctgcgcg gactgcaggt gatgcagctg     720
   gcggacgtgg tgctctacga tcatctcgtc agcgatgagg tgctcgatct ggtccggcgc     780
   gatgccgatc gtatctgcgt aggcaagcgt gccagcgccc atctcctgcc gcaggacgaa     840
   attaaccagt tgatggtgca actggcgcag aaaggcaaac gtgtggtgcg ccttaaaggc     900
   ggcgatccct ttatttttgg ccgcggcggc gaagagttac aggcggcggc gcaagcgggc     960
   attccattcc aggtcgtgcc tggcgtaacg gccgccgcag ggctaccgcc ctatgctggc     1020
   attccgctga cgcaccgtga ttacgcacaa agcgtgctgt ttatcaccgg acactgccgt    1080
   ccggatggcg atgatattga ctggccatcc ctcgcgcgtg cccgtcagac gctggcgatt    1140
   tacatgggcg ctgtcaaggc ggctcacatc agccagcagc ttattcttca tgggcgcgcc    1200
   gcctcaacac cggttgcggt gattgggcgc ggtacccggc cggatcaaca ggtattgacc    1260
   ggcacactcg aacatctgga gacgctggca gcgtcagcgc cttccccggc cctgctggtg    1320
   attggggaag tggttaattt acacgggcaa ctggcctggt ttcagcattc ggcacagcag    1380
   ggggctcgcg agtccgccgt tgtcaatctg gcttga                              1416
<210> 2
   <211> 912
   <212> DNA
   <213> Pantoea ananatis
<400> 2
   atggaccaaa aacgactcac tcatctgcgc caactggagg cggagagcat ccatattatc      60
   cgcgaagtgg cagccgaatt cggcaatccg gtcatgatgt attccatcgg taaggattcc     120
   tctgtcatgc tgcatctggc gcgtaaggcc ttctacccag gcacgttgcc gtttccgttg     180
   ctgcacgtgg acaccggctg gaaatttcgc gagatgtatg aatttcgcga ccgcacccgt     240
   caaagcgatg ggcgccggag ctgttggtac atcgcaattc ccgaaggggt ggcgatgggg     300
   atcaacccct ttgtgcacgg cagtgccaag cacaccgaca ttatgaaaac cgaaggcctg     360
   aagcaggcgc tgaacaagta cggctttgat gcggccttcg gcggcgcccg tcgcgatgaa     420
   gagaagtcac gtgccaaaga gcgtatttac tcgttccgtg accgttttca ccgctgggac     480
   ccaaaaaatc agcgcccgga actgtggcac aactataacg gccagattaa caaaggcgag     540
   agcatccgtg ttttcccgct gtctaactgg accgagctgg atatctggca atacatcttc     600
   ctggaaaaca ttgagatcgt gccgctttat ctggccgcgc cgcgtcctgt actggaacgt     660
   gatggcatgt tgatgatgat cgatgacgat cgtattaacc tccagccagg tgaagtgatc     720
   aaacagcgca tggttcgttt ccgtaccctt gggtgctggc cgttgaccgg tgcggtggaa     780
   tctgaagcgc aaaccttgcc tgagattatt gaagagatgc tggtttccac caccagcgag     840
   cgtcagggcc gtgtgattga ccgcgatcag tccggttcga tggaactgaa aaaacgtcag     900
   ggttatttct aa                                                         912
<210> 3
   <211> 1428
   <212> DNA
   <213> Pantoea ananatis
<400> 3
   atgaataccg taattgcaca acagattgcc gaacaggggg gcgttgaagc ctggctgacc       60
   gcacaacaac ataaaagcct gctgcgtttt ctgacctgcg gcagcgtcga tgacggcaaa      120
   agtacgctga ttggccgctt gctgcatgac acgcgtcaga tctatgaaga tcagctgtct      180
   tcattacaaa gcgacagcaa acgtcacggc acccagggcg agaaactgga tctggcgctg      240
   ctggtcgatg gtttacaggc cgagcgcgag cagggaatta ccattgatgt cgcctatcgc      300
   tatttctcca ccgaaaagcg taagttcatc atcgccgaca cgccaggcca tgagcagtac      360
   acccgcaata tggcaacggg cgcatcaacc tgtgacctgg cgatcctgct gatcgacgcc      420
   cgcaaaggcg tactggatca gacgcgtcgc cacagcttta tttcgacgct gctgggcatc      480
   aagcatctgg ttgtggcgat aaacaaaatg gatctggtgg attaccagca gtctgtgttt      540
   gagcagatca agcaggatta cctcgatttt gccggccagt tgcctgaaga cctggatatt      600
   cgctttgtgc cgatgtcggc gctggaaggc gaaaacgtcg cctcacagag ccagagcatg      660
   ccgtggtaca gcggcccgac gttactcgac gttctggaaa ccgtggagtt aaacgcggtg      720
   gtggatcacc agccgatgcg tttcccggtg cagtatgtta atcgtcccaa tctcgacttc      780
   cgcggttatg ccggtacgct ggcctctggc gtggtgaaag tgggacagcg cgttaaagtc      840
   ctgccttcag gcgtggagtc caccattgcc cgcattgtga cctttgacgg cgatctgcag      900
   gaagcgggag cgggcgaggc cattacgctg gtcctgaacg acgaaatcga tatcagccgt      960
   ggcgatctgt tagtggacgc cagcgccgag ctgcgcgccg ttcagtccgc caccgtcgat     1020
   gtggtgtgga tggctgagca gccgcttcag gccggacaga gctacgatgt aaaaatcgcc     1080
   ggtaagaaag cccgtgggcg agtggaaaag gtcattcacc aggttgagat taattcactg     1140
   gagaaacggc aggtggacaa tctgccgctg aacgggattg gccttgctga gtttacgttt     1200
   gatgagccgc tggtgcttga cagctatcag caaaatcccg taaccggcgg catgatcatt     1260
   attgaccgtc tgagtaacgt caccgtaggc gcggggctga ttcatgaacc actgaacgat     1320
   cggccgcagc acaatggcga gttcagcgcg ttcgaactcg agcttaatgc actggtccgt     1380
   cgccacttcc cgcactggaa tgcgcgcgat ctgctgggcg gtcagtaa                  1428
<210> 4
   <211> 606
   <212> DNA
   <213> Pantoea ananatis
<400> 4
   atggcccaac acgatgaaaa tgtagtatgg catgaccatc cggttacgca gcaggcacgc     60
   gaacagcaac atggccatca gggcgtggta ctgtggttta ccggcctttc ggggtccggt    120
   aaatccaccg ttgcgggggc gctggagcag gcgctacacc gtatcggtgt cagcacctat    180
   ctgctggacg gcgataatgt ccgtcacggg ttgtgtcgcg atctgggctt tagcgatgaa    240
   catcgtaaag agaatatccg ccgtgtaggc gaggtggcca aactgatggt ggatgccggt    300
   ttggtcgtcc ttaccgcctt tatctcaccg caccgtgccg agcgccagat ggtgcgcgat    360
   ctgctggaca gcgaccggtt tatcgaggtt tttgtcgata cgccgctggc agtgtgtgaa    420
   gcgcgcgatc ctaaagggtt gtacaaaaaa gcccgtgcag gcgagttaca gaatttcacc    480
   ggaatagaca gcgtttatga agcgccggac cagccagaaa tctggcttaa cggggaacaa    540
   ttggttacaa aactgacggc ccaattgtta gatctgctgc gtcagcgcga tattatcagt    600
   tcctga                                                               606
<210> 5
   <211> 756
   <212> DNA
   <213> Pantoea ananatis
<400> 5
   atgaggtgga aaatgttaga gcaaataagc caactggcgc gtgaagcagg cgacgcgatt     60
   atgcaggtct acaacggttc cgttccgaca gacgtttctc acaaagcgga tgattctcct    120
   gtcacggcgg cagacctggc cgctcatgat gtgattgtgt cgggcttaaa acagctgaca    180
   ccggatattc cggtgctgtc agaggaagat ccgcccggct gggaggttcg ccagcactgg    240
   caccgttact ggctggttga cccgctggat ggcaccaaag aatttatcaa acgtaacggc    300
   gaatttaccg tgaatattgc gctgattgag cagggcaaac cggtaatggg cgtggtttat    360
   gctcccgcgc tgggcgtgat gtattccgcc gcagagggca aagcctggaa agaagagaac    420
   ggcgagcgac aacagattca tgtgcttgat gcgcgtccac cgttagtggt ggtgagccgt    480
   tcgcacaacg acgacgacga gatgaaagag tacctcaagc aactggggga acatcagacg    540
   gtggcaaccg ggtcatcact gaagttttgc ctggtggcag aagggaaagc acagctctat    600
   ccgcgctttg ggccaaccaa tatttgggat accggagcgg gtcatgccgt tgccgcggcc    660
   gcgggtgctc acgttcacga ctggcaggga agaacactgg actatgcacc gcgtgaatct    720
   tttcttaatc ccggctttcg cgtctcgctg ttttaa                              756
<210> 6
   <211> 36
   <212> DNA
   <213> Artificial sequence
<220>
   <223> primer P1
<400> 6
   agctgagtcg acccccagga aaaattggtt aataac 36
<210> 7
   <211> 33
   <212> DNA
   <213> Artificial sequence
<220>
   <223> primer P2
<400> 7
   agctgagcat gcttccaact gcgctaatga cgc 33
<210> 8
   <211> 33
   <212> DNA
   <213> Artificial sequence
<220>
   <223> primer P3
<400> 8
   agctgatcta gaaaacagaa tttgcctggc ggc 33
<210> 9
   <211> 33
   <212> DNA
   <213> Artificial sequence
<220>
   <223> primer P4
<400> 9
   agctgaggat ccaggaagag tttgtagaaa cgc 33
<210> 10
   <211> 32
   <212> DNA
   <213> Artificial sequence
<220>
   <223> primer P5
<400> 10
   agctgagtcg acgtgttcgc tgaatacggg gt 32
<210> 11
   <211> 32
   <212> DNA
   <213> Artificial sequence
<220>
   <223> primer P6
<400> 11
   agctgatcta gagaaagcat caggattgca gc 32
<210> 12
   <211> 59
   <212> DNA
   <213> Artificial sequence
<220>
   <223> primer P7
<220>
   <221> misc_feature
   <222> (25)..(51)
   <223> "n"- any nucleotide
<400> 12
   atcgtgaaga tcttttccag tgttnannag ggtgccttgc acggtnatna ngtcactgg 59
<210> 13
   <211> 32
   <212> DNA
   <213> Artificial sequence
<220>
   <223> primer P8
<220>
   <221> misc_feature
   <222> (16)..(20)
   <223> "n"-any nucleotide
<400> 13
   tggaaaagat cttctnnnnn cgctgacctg cg 32
<210> 14
   <211> 60
   <212> DNA
   <213> Artificial sequence
<220>
   <223> primer P9
<400> 14
   tccgctcacg atttttttca tcgctggtaa ggtcatttat cccccaggaa aaattggtta 60
<210> 15
   <211> 66
   <212> DNA
   <213> Artificial sequence
<220>
   <223> primer P10
<400> 15
   tgcttcacgc gccagttggc ttatttgctc taacatagcc tgtccttaac tgtatgaatt 60
   attggg 66
<210> 16
   <211> 20
   <212> DNA
   <213> Artificial sequence
<220>
   <223> primer P11
<400> 16
   ctttgtccct ttagtgaagg 20
<210> 17
   <211> 44
   <212> DNA
   <213> Artificial sequence
<220>
   <223> primer P12
<400> 17
   agctgatcta gaagctgact cgagttaatg gcctcccaga cgac 44
<210> 18
   <211> 33
   <212> DNA
   <213> Artificial sequence
<220>
   <223> primer P13
<400> 18
   agctgagtcg acatggcaaa ggtatcactg gaa 33
<210> 19
   <211> 34
   <212> DNA
   <213> Artificial sequence
<220>
   <223> primer P14
<400> 19
   gagaacgccc gggcgggctt cgtgaatatg cagc 34
<210> 20
   <211> 32
   <212> DNA
   <213> Artificial sequence
<220>
   <223> primer P15
<400> 20
   agctgatcta gacgtgggat cagtaaagca gg 32
<210> 21
   <211> 22
   <212> DNA
   <213> Artificial sequence
<220>
   <223> primer P16
<400> 21
   aaaaccgccc gggcgttctc ac 22
<210> 22
   <211> 68
   <212> DNA
   <213> Artificial sequence
<220>
   <223> primer P17
<400> 22
   ggtcaacatt atggggaaaa actcctcatc ctttagcgtg tgaagcctgc ttttttatac 60
   taagttgg 68
<210> 23
   <211> 68
   <212> DNA
   <213> Artificial sequence
<220>
   <223> primer P18
<400> 23
   ttacttctgg tcgggcagcg cataggcaat cacgtaatcg cgctcaagtt agtataaaaa 60
   agctgaac 68
<210> 24
   <211> 64
   <212> DNA
   <213> Artificial sequence
<220>
   <223> primer P19
<400> 24
   acggtgatta cacaatttga atcgtccgga aagccttgaa gcctgctttt ttatactaag 60
   ttgg 64
<210> 25
   <211> 60
   <212> DNA
   <213> Artificial sequence
<220>
   <223> primer P20
<400> 25
   acctgcgaga tcggcaaaaa gaggcaaata atccacttat cccccaggaa aaattggtta 60
<210> 26
   <211> 19
   <212> DNA
   <213> Artificial sequence
<220>
   <223> primer P21
<400> 26
   tcggacatac ggctgaagc 19
<210> 27
   <211> 23
   <212> DNA
   <213> Artificial sequence
<220>
   <223> primer P22
<400> 27
   acggtgatta cacaatttga atc 23
<210> 28
   <211> 20
   <212> DNA
   <213> Artificial sequence
<220>
   <223> primer P38
<400> 28
   tgcgcctggt taagctggcg 20
<210> 29
   <211> 64
   <212> DNA
   <213> Artificial sequence
<220>
   <223> primer P24
<400> 29
   gcgttgcagg ctctgtaaaa ggatatcgtt gccatttgaa gcctgctttt ttatactaag 60
   ttgg 64
<210> 30
   <211> 36
   <212> DNA
   <213> Artificial sequence
<220>
   <223> primer P25
<400> 30
   actgcagtcg actccttaac tgtatgaatt attggg 36
<210> 31
   <211> 33
   <212> DNA
   <213> Artificial sequence
<220>
   <223> primer P26
<400> 31
   agctgagcat gcataacgtt ttgagtcagc cgc 33
<210> 32
   <211> 62
   <212> DNA
   <213> artificial sequence
<220>
   <223> forward terminator synthetic oligonucleotide
<400> 32
   aagcttaaca cagaaaaaag cccgcacctg acagtgcggg cttttttttt cgaccactgc 60
   ag 62
<210> 33
   <211> 62
   <212> DNA
   <213> artificial sequence
<220>
   <223> reveres terminator synthetic oligonucleotide
<400> 33
   ttcgaattgt gtcttttttc gggcgtggac tgtcacgccc gaaaaaaaaa gctggtgacg 60
   tc 62
<210> 34
   <211> 37
   <212> DNA
   <213> artificial sequence
<220>
   <223> primer
<400> 34
   ccagatcttg aagcctgctt ttttatacta agttggc 37
<210> 35
   <211> 25
   <212> DNA
   <213> artificial sequence
<220>
   <223> primer
<400>, 35
   gaaatcaaat aatgatttta ttttg 25
<210> 36
   <211> 26
   <212> DNA
   <213> artificial sequence
<220>
   <223> primer
<400> 36
   ttacgccccg ccctgccact catcgc 26
<210> 37
   <211> 34
   <212> DNA
   <213> artificial sequence
<220>
   <223> primer
<400> 37
   gtcactgcag ctgatgtccg gcggtgcttt tgcc 34
<210> 38
   <211> 31
   <212> DNA
   <213> artificial sequence
<220>
   <223> primer
<400> 38
   cagctgcagt ctgttacagg tcactaatac c 31
<210> 39
   <211> 37
   <212> DNA
   <213> artificial sequence
<220>
   <223> primer
<400> 39
   ccgagctccg ctcaagttag tataaaaaag ctgaacg 37
<210> 40
   <211> 70
   <212> DNA
   <213> artificial sequence
<220>
   <223> primer
<400> 40
   cccgagctcg gtacctcgcg aatgcatcta gatgggcccg tcgactgcag aggcctgcat 60
   gcaagcttcc 70
<210> 41
   <211> 120
   <212> DNA
   <213> DNA fragment, attL
<400> 41
   agatcttgaa gcctgctttt ttatactaag ttggcattat aaaaaagcat tgcttatcaa 60
   tttgttgcaa cgaacaggtc actatcagtc aaaataaaat cattatttga tttcgaattc 120
<210> 42
   <211> 40
   <212> DNA
   <213> Artificial sequence
<220>
   <223> primer P27
<400> 42
   ctagtaagat cttgaagcct gcttttttat actaagttgg 40
<210> 43
   <211> 41
   <212> DNA
   <213> Artificial sequence
<220>
   <223> primer P28
<400> 43
   atgatcgaat tcgaaatcaa ataatgattt tattttgact g 41
<210> 44
   <211> 184
   <212> DNA
   <213> DNA fragment, attR
<400> 44
   ctgcagtctg ttacaggtca ctaataccat ctaagtagtt gattcatagt gactgcatat    60
   gttgtgtttt acagtattat gtagtctgtt ttttatgcaa aatctaattt aatatattga   120
   tatttatatc attttacgtt tctcgttcag cttttttata ctaacttgag cgtctagaaa   180
   gctt                                                                 184
<210> 45
   <211> 41
   <212> DNA
   <213> Artificial sequence
<220>
   <223> primer P29
<400> 45
   atgccactgc agtctgttac aggtcactaa taccatctaa g 41
<210> 46
   <211> 46
   <212> DNA
   <213> Artificial sequence
<220>
   <223> primer P30
<400> 46
   accgttaagc tttctagacg ctcaagttag tataaaaaag ctgaac 46
<210> 47
   <211> 38
   <212> DNA
   <213> Artificial sequence
<220>
   <223> primer P31
<400> 47
   ttcttagacg tcaggtggca cttttcgggg aaatgtgc 38
<210> 48
   <211> 37
   <212> DNA
   <213> Artificial sequence
<220>
   <223> primer P32
<400> 48
   taacagagat ctcgcgcaga aaaaaaggat ctcaaga 37
<210> 49
   <211> 46
   <212> DNA
   <213> Artificial sequence
<220>
   <223> primer P33
<400> 49
   aacagagatc taagcttaga tcctttgcct ggcggcagta gcgcgg 46
<210> 50
   <211> 35
   <212> DNA
   <213> Artificial sequence
<220>
   <223> primer P34
<400> 50
   ataaactgca gcaaaaagag tttgtagaaa cgcaa 35
<210> 51
   <211> 1388
   <212> DNA
   <213> DNA fragment containing Tc gene and ter_thrL
<400> 51
   gaattctcat gtttgacagc ttatcatcga taagctttaa tgcggtagtt tatcacagtt      60
   aaattgctaa cgcagtcagg caccgtgtat gaaatctaac aatgcgctca tcgtcatcct     120
   cggcaccgtc accctggatg ctgtaggcat aggcttggtt atgccggtac tgccgggcct     180
   cttgcgggat atcgtccatt ccgacagcat cgccagtcac tatggcgtgc tgctagcgct     240
   atatgcgttg atgcaatttc tatgcgcacc cgttctcgga gcactgtccg accgctttgg     300
   ccgccgccca gtcctgctcg cttcgctact tggagccact atcgactacg cgatcatggc     360
   gaccacaccc gtcctgtgga tcctctacgc cggacgcatc gtggccggca tcaccggcgc     420
   cacaggtgcg gttgctggcg cctatatcgc cgacatcacc gatggggaag atcgggctcg     480
   ccacttcggg ctcatgagcg cttgtttcgg cgtgggtatg gtggcaggcc ccgtggccgg     540
   gggactgttg ggcgccatct ccttgcatgc accattcctt gcggcggcgg tgctcaacgg     600
   cctcaaccta ctactgggct gcttcctaat gcaggagtcg cataagggag agcgtcgacc     660
   gatgcccttg agagccttca acccagtcag ctccttccgg tgggcgcggg gcatgactat     720
   cgtcgccgca cttatgactg tcttctttat catgcaactc gtaggacagg tgccggcagc     780
   gctctgggtc attttcggcg aggaccgctt tcgctggagc gcgacgatga tcggcctgtc     840
   gcttgcggta ttcggaatct tgcacgccct cgctcaagcc ttcgtcactg gtcccgccac     900
   caaacgtttc ggcgagaagc aggccattat cgccggcatg gcggccgacg cgctgggcta     960
   cgtcttgctg gcgttcgcga cgcgaggctg gatggccttc cccattatga ttcttctcgc    1020
   ttccggcggc atcgggatgc ccgcgttgca ggccatgctg tccaggcagg tagatgacga    1080
   ccatcaggga cagcttcaag gatcgctcgc ggctcttacc agcctaactt cgatcactgg    1140
   accgctgatc gtcacggcga tttatgccgc ctcggcgagc acatggaacg ggttggcatg    1200
   gattgtaggc gccgccctat accttgtctg cctccccgcg ttgcgtcgcg gtgcatggag    1260
   ccgggccacc tcgacctgaa tggaagccgg cggcacctcg ctaacggatt caccactcca    1320
   actagaaagc ttaacacaga aaaaagcccg cacctgacag tgcgggcttt ttttttcgac     1380
   cactgcag                                                             1388
<210> 52
   <211> 36
   <212> DNA
   <213> Artificial sequence
<220>
   <223> primer P35
<400> 52
   agtaattcta gaaagcttaa cacagaaaaa agcccg 36
<210> 53
   <211> 43
   <212> DNA
   <213> Artificial sequence
<220>
   <223> primer P36
<400> 53
   ctagtaggat ccctgcagtg gtcgaaaaaa aaagcccgca ctg 43
<210> 54
   <211> 20
   <212> DNA
   <213> Artificial sequence
<220>
   <223> primer P37
<400> 54
   tgacaacaat ctatctgatt 20
<210> 55
   <211> 34
   <212> DNA
   <213> Artificial sequence
<220>
   <223> primer PrOMPCF
<400> 55
   agctgagtcg acaaccctct gttatatgcc ttta 34
<210> 56
   <211> 30
   <212> DNA
   <213> Artificial sequence
<220>
   <223> primer PrOMPCR
<400> 56
   agctgagcat gcgagtgaag gttttgtgac 30
<210> 57
   <211> 34
   <212> DNA
   <213> Artificial sequence
<220>
   <223> primer mz017
<400> 57
   agctgagtcg acatgttaga tcaagtatgc cagc 34
<210> 58
   <211> 35
   <212> DNA
   <213> Artificial sequence
<220>
   <223> primer mz018
<400> 58
   agctgatcta gactgaattt agtaaataga cactc 35
<210> 59
   <211> 34
   <212> DNA
   <213> Artificial sequence
<220>
   <223> primer N1
<400> 59
   agctgagtcg actccttaac tgtatgaaat tggg 34
<210> 60
   <211> 33
   <212> DNA
   <213> Artificial sequence
<220>
   <223> primer N2
<400> 60
   agctgagcat gcccagcctg tttacgatga tcc 33
<210> 61
   <211> 34
   <212> DNA
   <213> Artificial sequence
<220>
   <223> primer N3
<400> 61
   agctgagtcg acatggatca aatacgactt actc 34
<210> 62
   <211> 32
   <212> DNA
   <213> Artificial sequence
<220>
   <223> primer N4
<400> 62
   agctgatcta gaaaacccgg tggtgtctca gg 32
<210> 63
   <211> 33
   <212> DNA
   <213> Artificial sequence
<220>
   <223> primer N5
<400> 63
   agctgatcta gacagacgac gttttcgtca tgc 33
<210> 64
   <211> 33
   <212> DNA
   <213> Artificial sequence
<220>
   <223> primer N6
<400> 64
   agctgagtcg acatggcgct gcatgacgaa aac 33
<210> 65
   <211> 147
   <212> DNA
   <213> Artificial sequence
<220>
   <223> Promoter PnlpD
<400> 65
   aaaacgtgag gaaatacctg gatttttcct ggttattttg ccgcaggtca gcgtatcgtg 60
<210> 66
   <211> 147
   <212> DNA
   <213> Artificial sequence
<220>
   <223> Promoter Pnlp8
<400> 66
   aaaacgtgag gaaatacctg gatttttcct ggttattttg ccgcaggtca gcgtataatg 60
   aagatctttt ccagtgttga caagggtgcc ttgcacggtt ataatgtcac tggttattaa 120
   ccaatttttc ctgggggata aatgagc 147

## Description

### Technical Field

The present invention relates to the microbiological industry, and specifically to a method for producing an L-cysteine, L-cystine, a derivative or precursor thereof or a mixture thereof using a bacterium of *Enterobacteriaceae* family which has been modified to have enhanced expression of the genes involved in the process of sulphur assimilation.

### Background Art

Conventionally, L-amino acids are industrially produced by fermentation methods utilizing strains of microorganisms obtained from natural sources, or mutants thereof. Typically, the microorganisms are modified to enhance production yields of L-amino acids.

Many techniques to enhance L-amino acid production yields have been reported, including transformation of microorganisms with recombinant DNA (see, for example, US patent No. 4,278,765). Other techniques for enhancing production yields include increasing the activities of enzymes involved in amino acid biosynthesis and/or desensitizing the target enzymes to the feedback inhibition by the resulting L-amino acid (see, for example, WO 95/16042 or US patent Nos. 4,346,170; 5,661,012 and 6,040,160).

The synthesis of L-cysteine from inorganic sulphur is the predominant mechanism by which reduced sulphur is incorporated into organic compounds in microorganisms. In this process, inorganic sulphate, the most abundant source of utilizable sulphur in the aerobic biosphere, is taken up and reduced to sulfide, which is then incorporated into L-cysteine in a step that is equivalent to the fixation of ammonia into glutamine or glutamate. There are many genes involved in the process of sulphur assimilation including the genes involved in sulphate activation (*cysD*, *cysN*, *cysC*) and adenosine 3'-phosphate 5'-phosphosulphate (PAPS) degradation (*cysQ*).

Inorganic sulphate is reduced to sulphide by a sequence of enzymatic steps involving ATP sulphurylase (EC 2.7.7.4), adenylylsulphate (APS) kinase (EC 2.7.1.25), phospho-adenylylsulphate (PAPS) reductase, and sulphite reductase (EC 1.8.1.2. NADPH dependent, or EC 1.8.7.1 ferredoxin dependent). O-acetyl-L-serine (thiol) lyase (EC 4.2.99.8) incorporates the sulphide forming the amino acid cysteine (Krone F.A. et.al., Mol.Gen.Genet., 225(2): 314-9 (1991)).

The DNA sequence of the sulphate activation locus from *E. coli* K-12 has been determined. The sequence includes the structural genes which encode the enzymes ATP sulfurylase (*cysD* and *cysN*) and APS kinase (*cyst*), which catalyze the synthesis of activated sulphate. These are the only genes known to be present on the sulphate activation operon. Consensus elements of the operon promoter were identified, and the start codons and open reading frames of the Cys polypeptides were determined. The activity of ATP sulfurylase is stimulated by an intrinsic GTPase. Comparison of the primary sequences of CysN and Ef-Tu revealed that many of the residues integral to the three-dimensional structure important for guanine nucleotide binding in Ef-Tu and RAS are conserved in CysN. *nodP* and *nodQ*, both from *Rhizobium meliloti*, are essential for nodulation in leguminous plants. The Cys and Nod proteins are remarkably similar. NodP appears to be the smaller subunit of ATP sulfurylase. *nodQ* encodes homologues of both CysN and CysC; thus, these enzymes may be covalently associated in *R. meliloti.* The consensus GTP-binding sequences of NodQ and CysN are identical, suggesting that NodQ encodes a regulatory GTPase (Leyh T.S. et.al., J.Biol.Chem., 267(15): 10405-10 (1992)).

The initial steps in assimilation of sulphate during cysteine biosynthesis entail sulphate uptake and sulphate activation by the formation of adenosine 5'-phosphosulphate, conversion to 3'-phosphoadenosine 5'-phosphosulphate, and reduction to sulfite. Mutations in the *Escherichia coli cysQ* gene, which resulted in a requirement for sulfite or cysteine, were obtained by *in vivo* insertion of transposons Tn5tac1 and Tn5supF, and by *in vitro* insertion of resistance gene cassettes. *cysQ* is at chromosomal position 95.7 min (kb 4517 to 4518) and is transcribed divergently from the adjacent *cpdB* gene. A Tn5tac1 insertion just inside the 3' end of *cysQ,* with its isopropyl-beta-D-thiogalactopyranoside-inducible *tac* promoter pointed toward the *cysQ* promoter, resulted in auxotrophy only when isopropyl-beta-D-thiogalactopyranoside was present; this conditional phenotype was ascribed to collision between converging RNA polymerases or interaction between complementary antisense and *cysQ* mRNAs. The auxotrophy caused by *cysQ* null mutations was leaky in some but not all *E. coli* strains and could be compensated by mutations in unlinked genes. *cysQ* mutants were prototrophic during anaerobic growth. Mutations in *cysQ* did not affect the rate of sulphate uptake or the activities of ATP sulfurylase and its protein activator, which together catalyze adenosine 5'-phosphosulphate synthesis. Some mutations that compensated for *cysQ* null alleles resulted in sulphate transport defects. *cysQ* is identical to a gene called *amtA*, which had been thought to be needed for ammonium transport. Computer analyses revealed significant amino acid sequence homology between *cysQ* and *suhB* of *E. coli* and the gene for mammalian inositol monophosphatase. Previous work had suggested that 3'-phosphoadenoside 5'-phosphosulphate is toxic if allowed to accumulate, and it is proposed that CysQ helps control the pool of 3'-phosphoadenoside 5'-phosphosulphate, or its use in sulfite synthesis (Neuwald A.F. et.al., J.Bacteriol., 174(2):415-25 (1992)).

A process for the preparation of L-threonine by fermentation of microorganisms of the *Enterobacteriaceae* family in which at least one or more of the genes of cysteine biosynthesis, such as *cysG, cysB, cysZ, cysK, cysM, cysA, cysW, cysU, cysP, cysD, cysN, cysC, cysJ, cysI, cysH, cysE* and *sbp*, is/are enhanced, in particular over-expressed, was disclosed (WO03006666A2).

A method for producing L-cysteine using a bacterium belonging to the genus *Escherichia* wherein the L-amino acid productivity of said bacterium is enhanced by enhancing expression of the genes of *cysPTWAM* cluster was disclosed (US2005124049A1).

But currently, there have been no reports of sequences of genes involved in the process of sulphur assimilation in bacteria of *Enterobacteriaceae* family and the enhancing expression of the genes for the purpose of producing L-cysteine using a bacterium of *Enterobacteriaceae* family.

### Disclosure of the Invention

Aspects of the present invention include enhancing the productivity of L-cysteine-producing strains and providing a method for producing L-cysteine, L-cystine, their derivatives or precursors, or a mixture of these using these strains.

The above aspects were achieved by the finding that enhancing expression of the genes involved in the process of sulphur assimilation can enhance production of L-cysteine.

The present invention uses a bacterium of of *Enterobacteriaceae* family having an increased ability to produce L-cysteine.

It is an aspect of the present invention to use an L-cysteine producing bacterium of *Enterobacteriaceae* family, wherein the bacterium has been modified to have enhanced expression of one or more genes involved in the process of sulphur assimilation.

It is a further aspect of the present invention to use the bacterium as described above, wherein the genes involved in the process of sulphur assimilation comprise the genes involved in sulphate activation and adenosine 3'-phosphate 5'-phosphosulphate (PAPS) degradation.

It is a further aspect of the present invention to use the bacterium as described above, wherein the genes involved in sulphate activation comprise one or more genes of *cysDNC* cluster.

It is a further aspect of the present invention to use the bacterium as described above, wherein the genes involved in adenosine 3'-phosphate 5'-phosphosulphate (PAPS) degradation comprise the gene *cysQ.*

It is a further aspect of the present invention to use the bacterium as described above, wherein the bacterium has been modified to have enhanced expression of *cysQ* gene, one or more genes of *cysDNC* cluster, or both

It is a further aspect of the present invention to use the bacterium as described above, wherein the expression of said gene(s) is/are enhanced by modifying an expression control sequence so that the expression of the gene(s) is/are enhanced.

It is a further aspect of the present invention to use the bacterium as described above, wherein native promoter(s) of said gene(s) is/are substituted with more potent promoter(s).

It is a further aspect of the present invention to use the bacterium as described above, wherein the bacterium belongs to the genus *Pantoea.*

It is a further aspect of the present invention to use the bacterium as described above, wherein the bacterium is *Pantoea ananatis.*

It is a further aspect of the present invention to use the bacterium as described above, wherein the bacterium belongs to the genus *Escherichia.*

It is a further aspect of the present invention to use the bacterium as described above, wherein the bacterium is *Escherichia coli.*

It is a further aspect of the present invention to provide a method for producing a compound selected from the group consisting of L-cysteine, L-cystine, derivatives and precursors thereof, which comprises cultivating the bacterium as described above in a culture medium containing sulphate, and collecting from the compound from the culture medium.

It is a further aspect of the present invention to provide the method as described above, wherein the bacterium has enhanced expression of genes involved in the biosynthesis of L-cysteine.

It is a further aspect of the present invention to provide the method as described above, wherein the bacterium has enhanced expression of genes involved in the biosynthesis of L-methionine.

### Brief Description of Drawings

Figure 1 shows sequences of native promoter P*_{nlpD}* (SEQ ID NO: 65) and modified promoter P*ₙₗₚ₈* (SEQ ID NO: 66).
Figure 2 shows construction of plasmid pM12.
Figure 3 shows construction of plasmid pM12-ter(thr).
Figure 4 shows construction of integrative cassette intJS.
Figure 5 shows construction of plasmid pMIV-5JS.

### Detailed Description of the Preferred Embodiments

The present invention is described in detail below.

### 1. Bacterium

The bacterium according to the presently disclosed subject matter can be an L-cysteine -producing bacterium of *Enterobacteriaceae* family, wherein the bacterium has been modified to have enhanced expression of the genes involved in the process of sulphur assimilation.

"L-cysteine-producing bacterium" can mean a bacterium which has an ability to produce and excrete an L-cysteine into a medium, when the bacterium is cultured in the medium.

The term "L-cysteine-producing bacterium" also can mean a bacterium which is able to produce and cause accumulation of an L-cysteine in a culture medium in an amount larger than a wild-type, unmodified, or parental strain, for example, *Pantoea ananatis,* such as *Pantoea ananatis* (*Enterobacter agglomerans*) strains AJ13355 (FERM BP-6614), AJ13356 (FERM BP-6615), AJ13601 (FERM BP-7207), SC17(US patent 7,090,998), or *Escherichia coli,* such as *E. coli* K-12. The SC17 strain is selected as a low phlegm-producing mutant strain from the AJ13355 strain isolated from soil in Iwata-shi, Shizuokaken, Japan as a strain that can proliferate in a low pH medium containing L-glutamic acid and a carbon source (U.S. Patent No. 6,596,517). The SC17 strain was deposited at the National Institute of Advanced Industrial Science and Technology, International Patent Organism Depository (address: AIST Tsukuba Central 6, 1-1, Higashi 1-Chome, Tsukubashi, Ibaraki-ken, 305-8566, Japan) on February 4, 2009, and assigned an accession number of FERM BP-11091. The term "L-cysteine-producing bacterium" can also mean that the microorganism is able to cause accumulation in a medium of an amount not less than 0.5 g/L, and in another example, not less than 1.0 g/L, of L-cysteine.

The L-cysteine produced by the bacterium can change into L-cystine in the medium by the formation of a disulfide bond. Especially, as described later, it is considered that if L-cysteine is produced in a large amount with by enhancing the DsbA activity, formation of L-cystine from L-cysteine by the DsbA-DsbB-UQ oxidation system is promoted. Furthermore, S-sulfocysteine can be generated by the reaction of L-cysteine and thiosulphuric acid in the medium (Szczepkowski T.W., Nature, vol. 182 (1958)). Furthermore, the L-cysteine generated in bacterial cells can be condensed with a ketone, aldehyde, or, for example, pyruvic acid, which is present in the cells, to produce a thiazolidine derivative via a hemithioketal intermediate (refer to Japanese Patent No. 2992010). This thiazolidine derivative and hemithioketal can be present as an equilibrated mixture. Therefore, the L-cysteine-producing ability is not limited to the ability to accumulate only L-cysteine in the medium or cells, but also includes the ability to accumulate L-cystine or its derivatives or precursors, or a mixture thereof in the medium.

Examples of the aforementioned derivative of L-cysteine or L-cystine include, for example, S-sulfocysteine, thiazolidine derivatives, hemithioketal, L-methionine, S-adenosylmethionine, and so forth. L-Cysteine is the precursor for L-methionine. L-Cysteine is involved in L-methionine biosynthesis in the reaction of conversion of O-succinyl-L-homoserine into L-cystathionine. So, increased level of L-cysteine may lead to increased accumulation of L-methionine. In turn, L-methionine is used as a starting material for synthesizing S-adenosylmethionine, and so forth. Therefore, if a bacterium has an ability to produce L-methionine or adenosylmethionine in addition to L-cysteine-producing ability, production of compounds such as L-methionine or adenosylmethionine can be enhanced by enhancing expression of the genes involved in the process of sulphur assimilation.

Examples of L-methionine-producing bacteria and parent strains which can be used to derive L-methionine-producing bacteria include, but are not limited to, *Escherichia* bacteria strains, such as strains AJ11539 (NRRL B-12399), AJ11540 (NRRL B-12400), AJ11541 (NRRL B-12401), AJ 11542 (NRRL B-12402) (patent GB2075055); strains 218 (VKPM B-8125) (patent RU2209248) and 73 (VKPM B-8126) (patent RU2215782) resistant to norleucine, the L-methionine analog, or the like.

Examples of the precursor of L-cysteine or L-cystine include, for example, O-acetylserine, which is a precursor of L-cysteine. The precursors of L-cysteine or L-cystine also include derivatives of the precursors, and examples include, for example, N-acetylserine, which is a derivative of O-acetylserine, and so forth.

O-Acetylserine (OAS) is a precursor of L-cysteine biosynthesis. OAS is a metabolite of bacteria and plants, and is produced by acetylation of L-serine by an enzymatic reaction catalyzed by serine acetyltransferase (SAT). OAS is further converted into L-cysteine in cells.

The L-cysteine-producing bacterium can inherently have the ability to produce L-cysteine, or it can be imparted by modifying a microorganism such as those described below by mutagenesis or a recombinant DNA technique. Unless specially mentioned, the term L-cysteine refers to the reduced-type L-cysteine, L-cystine, a derivative or precursor such as those mentioned above, or a mixture thereof.

The bacterium is not particularly limited so long as the bacterium belongs to the family *Enterobacteriaceae* and has L- cysteine-producing ability. The family *Enterobacteriaceae* encompasses bacteria belonging to the genera *Escherichia, Enterobacter, Erwinia, Klebsiella, Pantoea, Photorhabdus, Providencia, Salmonella, Serratia, Shigella, Morganella, Yersinia*, and so forth. Particular examples include bacteria classified into the family *Enterobacteriaceae* according to the taxonomy used by the NCBI (National Center for Biotechnology Information) database (http://www.ncbi.nlm.nih.gov/TaxonomyBrowser/wwwtax.cgi?id=91347).

The phrase "a bacterium belonging to the genus *Pantoea"* can mean that the bacterium is classified as the genus *Pantoea* according to the classification known to a person skilled in the art of microbiology. Some species of *Enterobacter agglomerans* have been recently re-classified into *Pantoea agglomerans, Pantoea ananatis, Pantoea stewartii* or the like, based on the nucleotide sequence analysis of 16S rRNA, etc. (Int. J. Syst. Bacteriol., 43, 162-173 (1993)).

A "bacterium belonging to the genus *Escherichia"* can mean that the bacterium is classified into the genus *Escherichia* according to the classification known to a person skilled in the art of microbiology, although the bacterium is not particularly limited. Examples of the bacterium belonging to the genus *Escherichia* include, but are not limited to, *Escherichia coli* (*E. coli*).

Examples of L-cysteine-producing bacteria and parent strains which can be used to derive L-cysteine-producing bacteria include, but are not limited to, *Escherichia* bacteria strains, such as *E. coli* JM15 which is transformed with different *cysE* alleles coding for feedback-resistant serine acetyltransferases (U.S. Patent No. 6,218,168, Russian patent application 2003121601), *E. coli* W3110 which over-expresses genes encoding proteins which direct the secretion of substances which are toxic to cells (U.S. Patent No. 5,972,663), *E. coli* strains with reduced cysteine desulfohydrase activity (JP 11155571 A2), *E. coli* W3110 with increased activity of a positive transcriptional regulator for the cysteine regulon encoded by the *cysB* gene (WO01/27307A1), and so forth.

The term "process of sulphur assimilation" can mean a process by which environmental sulphur, for example sulphate, is fixed into organic sulphur for use in cellular metabolism. The two major end products of this process are the essential amino acids L-cysteine and L-methionine. Key to that process is increasing the level of organic sulphur available for L-cysteine and L-methionine biosynthesis.

Examples of "one or more genes involved in the process of sulphur assimilation and adenosine 3'-phosphate 5'-phosphosulphate (PAPS) degradation" include *cysG*, *cysD*, *cysN*, *cysC*, *cysQ* and combinations thereof. The *cysG*, *cysD*, *cysN*, *cysC*, *cysQ* are involved in sulphur assimilation, and the *cysQ* is involoved PAPS degradation. Examples of "one or more genes involved in the process of sulphur assimilation and PAPS degradation includes *cysD*, *cysN and cysC*, or *cysQ* alone, combination of *cysD* and *cysN*, combination of *cysD*, *cysN* and *cysC,* combination of *cysD*, *cysN* and *cysQ*, and commbination of *cysD*, *cysN*, *cysC* and *cysQ*.

The system for sulphate activation of *E. coli* is known. The system includes the structural genes encoding the enzymes ATP sulfurylase (*cysD* and *cysN*) and APS kinase (*cysC*). The initial steps in assimilation of sulphate during cysteine biosynthesis entail sulphate uptake and sulphate activation by formation of adenosine 5'-phosphosulphate, conversion to 3'-phosphoadenosine 5'-phosphosulphate, and reduction to sulfite. The *cysQ* gene is involved in the process. The *cysG* gene of *E. coli* encodes the protein CysG, which is a subunit of uroporphyrin III C-methyltransferase / precorrin-2 dehydrogenase / sirohydrochlorin ferrochelatase. The *cysD* gene of *E*. *coli* encodes the protein CysD-component of sulphate adenylyltransferase. The *cysN* gene *of E. coli* encodes the protein CysN-component of sulphate adenylyltransferase. The *cysC* gene *of E. coli* encodes the protein CysC-subunit composition of adenylylsulphate kinase. The *cysQ* gene of *E. coli* encodes the protein CysQ which is proposed to help control the pool of 3'-phosphoadenoside 5'-phosphosulphate, or its use in sulfite synthesis. In *Escherihica coli*, *cysD*, *cysN* and *cysC* genes forms *cysDNC* operon.

Genes from *P. ananatis* which are homologous to *E. coli* genes of sulphur utilization system were found and cloned. The nucleotide sequence of the *cysG* gene of *P. ananatis* is shown in SEQ ID NO: 1. The nucleotide sequence of the *cysD* gene of *P. ananatis* is shown in SEQ ID NO: 2. The nucleotide sequence of the *cysN* gene of *P. ananatis* is shown in SEQ ID NO: 3. The nucleotide sequence of the *cysC* gene of *P. ananatis* is shown in SEQ ID NO: 4. The nucleotide sequence of the *cysQ* gene of *P. ananatis* is shown in SEQ ID NO: 5.

In *Pantea ananatis*, *cysG*, *cysD*, *cysN* and *cysC* genes forms *cysGDNC* operon. Enhancing expression of *cysG* is not esssential. However, expression of *cysG* can be enhanced.

Since there may be some differences in DNA sequences between the genera or strains of the genus *Pantoea*, the genes *cysG*, *cysD*, *cysN*, *cysC* and *cysQ* are not limited to the genes shown in SEQ ID NO: 1, SEQ ID NO: 2, SEQ ID NO: 3, SEQ ID NO: 4 and SEQ ID NO: 5, respectively, but may include genes homologous to SEQ ID NO: 1, SEQ ID NO: 2, SEQ ID NO: 3, SEQ ID NO: 4 and SEQ ID NO: 5, respectively.

Therefore, the genes *cysG*, *cysD*, *cysN*, *cysC* and *cysQ* can each be a variant which hybridizes under stringent conditions with the nucleotide sequence complementrary to the nucleotide sequence shown in SEQ ID NO: 1, SEQ ID NO: 2, SEQ ID NO: 3, SEQ ID NO: 4 or SEQ ID NO: 5, or a probe which can be prepared from the nucleotide sequence. "Stringent conditions" include those under which a specific hybrid, for example, a hybrid having homology of not less than 60%, in another example not less than 70%, in another example not less than 80%, in another example not less than 90%, in another example not less than 95%, in another example not less than 98%, and in another example not less than 99%, is formed and a non-specific hybrid, for example, a hybrid having homology lower than the above, is not formed. For example, stringent conditions can be exemplified by washing one time or more, in another example two or three times at a salt concentration of 1×SSC, 0.1% SDS, in another example 0.1× SSC, 0.1% SDS at 60°C. Duration of washing depends on the type of membrane used for blotting and, as a rule, can be what is recommended by the manufacturer. For example, the recommended duration of washing for the Hybond^{™} N⁺ nylon membrane (Amersham) under stringent conditions is 15 minutes. Washing can be performed 2 to 3 times. The length of the probe can be suitably selected, depending on the hybridization conditions, and is usually 100 bp to 1 kbp.

The phrase "bacterium has been modified to have enhanced expression of the gene" can mean that the expression of the gene of a modified bacterium is higher than that of a non-modified strain, for example, a wild-type strain. Examples of such modification include increasing the copy number of expressed gene(s) per cell, increasing the expression level of the gene(s), and so forth. The quantity of the copy number of an expressed gene is measured, for example, by restricting the chromosomal DNA followed by Southern blotting using a probe based on the gene sequence, fluorescence in situ hybridization (FISH), and the like. The level of gene expression can be measured by various known methods including Northern blotting, quantitative RT-PCR, and the like. Furthermore, wild-type strains that can act as a control include, for example, *Pantoea ananatis* FERM BP-6614.

The term "expression" can mean the production of the protein product encoded by a gene.

The phrase "expression control sequence" refers to nucleotide sequences located upstream, within, and/or downstream of a coding region, and which control transcription and/or expression of the coding region in conjunction with the protein biosynthetic apparatus of the cell. The phrase is usually used when describing promoters, ribosome binding sites (RBS), operators, or other elements of a genome, and other elements which influence gene expression levels.

Furthermore, the enhancement of gene expression can be achieved by locating the DNA according to the presently disclosed subject matter under the control of a promoter more potent than the native promoter. The term "native promoter" means a DNA region which is present in the wild-type organism, is located upstream of the opened reading frame (ORF) of the gene or cluster of genes, and promotes expression of the gene/ cluster of genes. The strength of the promoter is defined by the frequency of acts of the RNA synthesis initiation. For example, the P*_{lac}* promoter, the P*ₜᵣₚ* promoter, the P*_{trc}* promoter, and the P_{R} or the P_{L} promoters of lambda phage are known as potent promoters. Examples of methods for evaluating strength of a promoter and strong promoters are described in the paper of Goldstein et al. (Prokaryotic promoters in biotechnology, Biotechnol. Annu. Rev., 1, 105-128 (1995)) and so forth. Enhancement of gene expression can also be achieved by placing a potent terminator downstream of the DNA according to the presently disclosed subject matter.

Furthermore, in addition to the aforementioned methods, gene expression can be enhanced by increasing the copy number of the gene by using, for example, a gene recombination technique. For example, a recombinant DNA can be prepared by ligating a gene fragment containing the target gene with a vector functioning in a host bacterium such as a multi-copy type vector and introduced into the bacterium to transform it. Examples of the vector include vectors which are autonomously replicable in host bacterium cells.

To introduce such a recombinant DNA into a bacterium, any known transformation methods that have hitherto been reported can be employed. For instance, employable are treating recipient cells with calcium chloride so as to increase permeability thereof for DNA, which has been reported for *Escherichia coli* K-12 (Mandel, M. and Higa, A., J. Mol. Biol., 53, 159 (1970)), and preparing competent cells from cells which are at the growth phase followed by introducing the DNA thereinto, which has been reported for *Bacillus subtilis* (Duncan, C.H., Wilson, G.A. and Young, F.E., Gene, 1, 153 (1977)). In addition to these, also employable is making DNA-recipient cells into protoplasts or spheroplasts, which can easily take up recombinant DNA, followed by introducing the recombinant DNA into the cells, which is known to be applicable to *Bacillus subtilis*, actinomycetes and yeasts (Chang, S. and Choen, S.N., Mol. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci. USA, 75, 1929 (1978)). In addition, microorganisms can also be transformed by electroporation (Japanese Patent Laid-open No. 2-207791).

Increase of the copy number of the gene can also be achieved by introducing multiple copies of the gene into a genomic DNA of a bacterium. In order to introduce multiple copies of the gene into a genomic DNA of a bacterium, homologous recombination is carried out by using a sequence whose multiple copies are present in the genomic DNA as targets. As sequences whose multiple copies are present in genomic DNA, repetitive DNA, and inverted repeats existing at the end of a transposable element can be used. Another target gene can be introduced aside the target gene existing on a genome in tandem, or it can be introduced into an unnecessary gene on a genome in a plural number. Such gene transfer can be attained by using a temperature sensitive vector or an integration vector.

Alternatively, as disclosed in Japanese Patent Laid-open No. 2-109985, it is also possible to incorporate the gene into a transposon, and allow it to transfer to introduce multiple copies of the genes into a genomic DNA. Transfer of the gene to the genome can be confirmed by performing Southern hybridization using a part of the gene as a probe.

Genes involved in the biosynthesis of L-cysteine can include different *cysE* alleles coding for feedback-resistant serine acetyltransferases (U.S. Patent No. 6,218,168, Russian patent application 2003121601); genes which encode proteins suitable for secreting substances toxic for cells (U.S. Patent No. 5,972,663); the *cysB* gene encoding a positive transcriptional regulator for cysteine regulon (WO0127307A1). Another example is for L-cysteine production to decrease the activity of cysteine desulfohydrase (JP11155571A2).

Genes involved in the biosynthesis of L-methionine can include genes of methionine regulon. The methionine regulon may have mutated genes coding for proteins lowered in activity in repressing the amino acid biosynthesis. Such gene is exemplified by variation type *metJ* gene coding for a L-methionine biosynthesis-relating repressor protein from *E. coli* of which activity in repressing methionine biosynthesis is lowered (JP2000157267A2).

Methods for preparation of plasmid DNA, digestion and ligation of DNA, transformation, selection of an oligonucleotide as a primer and the like are well known to one skilled in the art. These methods are described, for instance, in Sambrook, J., Fritsch, E.F., and Maniatis. T., "Molecular Cloning A Laboratory Manual, Second Edition"; Cold Spring Harbor Laboratory Press (1989).

### 2. Method of the present invention.

The method of the present invention is a method for producing a compound selected from the group consisting of L-cysteine, L-cystine, derivatives and precursors thereof, and mixture thereof, which includes the steps of cultivating the bacterium according to the presently disclosed subject matter in a culture medium to cause accumulation of the compound in the medium, and collecting the compound from the medium. Examples of a derivative or precursor of L-cysteine include S-sulfocysteine, a thiazolidine derivative, a hemithioketal corresponding the thiazolidine derivative mentioned above, O-acetylserine, N-acetylserine, and so forth.

The cultivation, collection, and purification of the compound from the medium and the like may be performed in a manner similar to conventional fermentation methods wherein a compound such as an amino acid is produced using a bacterium.

The medium which can be used in the culture can be either a synthetic or natural medium, so long as the medium includes a carbon source, a nitrogen source, minerals and, if necessary, appropriate amounts of nutrients which the bacterium may require for growth. The carbon source can include various carbohydrates such as glucose and sucrose, and various organic acids. Depending on the assimilation mode of the chosen microorganism, alcohol including ethanol and glycerol can be used. The nitrogen source can include various ammonium salts such as ammonia and ammonium sulphate, other nitrogen compounds such as amines, a natural nitrogen source such as peptone, soybean-hydrolysate, and digested fermentative microorganism. The sulphur source can include ammonium sulphate, magnesium sulphate, ferrous sulphate, manganese sulphate, and the like. Minerals can include potassium monophosphate, sodium chloride, calcium chloride, and the like. Vitamins can include thiamine, yeast extract, and the like.

The cultivation can be performed under aerobic conditions such as a shaking culture or a stirring culture with aeration, at a temperature of 20 to 40°C, and in another example 30 to 38°C. The pH of the culture is usually between 5 and 9, and in another example between 6.5 and 7.2. The pH of the culture can be adjusted with ammonia, calcium carbonate, various acids, various bases, and buffers. Usually, a 1 to 5-day cultivation period leads to the accumulation of the target compound in the liquid medium.

After cultivation, solids such as cells can be removed from the liquid medium by centrifugation or membrane filtration, and the target compound can be collected and purified by ion-exchange, concentration, and crystallization methods.

L-cysteine obtained as described above can be used to produce L-cysteine derivatives. The cysteine derivatives include methylcysteine, ethylcysteine, carbocysteine, sulfocysteine, acetylcysteine, and so forth.

Furthermore, when a thiazolidine derivative of L-cysteine is produced in the medium, L-cysteine can be produced by collecting the thiazolidine derivative from the medium to break the reaction equilibrium between the thiazolidine derivative and L-cysteine so that L-cysteine is excessively produced. Furthermore, when S-sulfocysteine is produced in the medium, it can be converted into L-cysteine by reduction with a reducing agent such as dithiothreitol.

### Examples

The present invention will be more concretely explained below with reference to the following non-limiting Examples.

### Example 1.Construction of a strain with enhanced expression of the genes of cysGDNC cluster

### 1. Construction of the strain P. ananatis EYPSG8

The DNA fragment containing the promoter of the *nlpD* gene from *E. coli* was obtained using PCR. The chromosomal DNA of *E. coli* MG1655 strain was used as a template, and primers P1 (SEQ ID No:6) and P2 (SEQ ID No:7) were used for PCR. The strain MG1655 (ATCC 47076) is available from American Type Culture Collection (Address: 12301 Parklawn Drive, Rockville, Maryland 20852, P.O. Box 1549, Manassas, VA 20108, United States of America). Conditions for PCR were as follows: denaturation step for 3 min at 95°C; profile for two first cycles: 1 min at 95°C, 30 sec at 50°C, 40 sec at 72°C; profile for the last 25 cycles: 20 sec at 94°C, 20 sec at 55°C, 15 sec at 72°C; final step: 5 min at 72°C. The amplified DNA fragment was about 0,2kb in size, and was purified by agarose gel electrophoresis. Then, the purified fragment was treated with endonucleases *Pae*I and *Sal*I*.* The obtained DNA fragment was then ligated with the plasmid pMIV-5JS (construction of the plasmid pMIV-5JS is described in Reference example 1) which had been previously treated with endonucleases *Pae*I and *Sal*I*.* The mixture for ligation was incubated at 4°C overnight and was then used to transform *E. coli* MG1655 strain by electroporation. The resulting transformants were plated on plates with LB agar containing ampicillin (50mg/l), and the plates were incubated at 37°C overnight until individual colonies were visible. Plasmids were isolated from the obtained transformants and analyzed by restriction analysis. The obtained plasmid contains the promoter of the *nlpD* gene from *E. coli,* and was named pMIV-Pnlp0.

The DNA fragment containing the terminator of the *rrnB* gene from *E. coli* was obtained using PCR. The chromosomal DNA of *E. coli* MG1655 strain was used as a template, and primers P3 (SEQ ID No:8) and P4 (SEQ ID No:9) were used for PCR. Conditions for PCR were as follows: denaturation step for 3 min at 95°C; profile for two first cycles: 1 min at 95°C, 30 sec at 50°C, 40 sec at 72°C; profile for the last 25 cycles: 20 sec at 94°C, 20 sec at 59°C, 15 sec at 72°C; final step: 5 min at 72°C. The amplified DNA fragment was about 0,3kb in size, and was purified by agarose gel electrophoresis. Then, the purified fragment was treated with endonucleases *BamH*I and *Xba*I*.* The obtained DNA fragment was then ligated with plasmid pMIV-PnIp0 which had been previously treated with endonucleases *BamH*I and *Xba*I. The mixture for ligation was incubated at 4°C overnight and was then used to transform *E. coli* MG1655 strain by electroporation. The resulting transformants were plated on plates with LB agar containing ampicillin (50mg/l), and the plates were incubated at 37°C overnight until individual colonies were visible. Plasmids were isolated from the obtained transformants and analyzed by restriction analysis. The obtained plasmid containing the terminator of the *rrnB* gene from *E. coli* was named pMIV-Pnlp0-ter.

The DNA fragment containing the *yeaS* gene from *E. coli* was obtained using PCR. The chromosomal DNA *of E. coli* MG1655 strain was used as a template, and primers P5 (SEQ ID No:10) and P6 (SEQ ID No:11) were used for PCR. Conditions for PCR were as follows: denaturation step for 3 min at 95°C; profile for two first cycles: 1 min at 95°C, 30 sec at 50°C, 40 sec at 72°C; profile for the last 25 cycles: 20 sec at 94°C, 20 sec at 55°C, 15 sec at 72°C; final step: 5 min at 72°C. The amplified DNA fragment was about 0,7kb in size, and was purified by agarose gel electrophoresis. Then, the purified fragment was treated with endonucleases *Sal*I and *Xba*I*.* The obtained DNA fragment was then ligated with the plasmid pMIV-Pnlp0-ter which had been previously treated with endonucleases *Sal*I and *Xba*I*.* The mixture for ligation was incubated at 4°C overnight, and was then used to transform the *E. coli* MG1655 strain by electroporation. The resulting transformants were plated on plates with LB agar containing ampicillin (50mg/l), and the plates were incubated at 37°C overnight until individual colonies were visible. Plasmids were isolated from the obtained transformants and analyzed by restriction analysis. The obtained plasmid containing the *yeaS* gene from *E. coli* was named pMIV-Pnlp0-yeaS3.

Then randomization of the -10 region of the promoter P*_{nipD}* and the selection of the P*ₙₗₚ₈* promoter was performed. The 3'-end of the promoter P*_{nlpD}* was obtained using PCR amplification. The plasmid pMIV-Pnlp0 was used as a template, and primers P1 SEQ ID No:6) and P7 (SEQ ID No: 12) were used for PCR. Primer P7 has random nucleotides, which are depicted in SEQ ID NO: 12 by the letter "n" (for A or G or C or T). Conditions for PCR were as follows: denaturation step for 3 min at 95°C; profile for two first cycles: 1 min at 95°C, 30 sec at 50°C, 40 sec at 72°C; profile for the last 25 cycles: 20 sec at 94°C, 20 sec at 60°C, 15 sec at 72°C; final step: 5 min at 72°C. 5'-end of promoter P*_{nlpD}* was obtained using PCR amplification. The plasmid pMIV-Pnlp0 was used as a template, and primers P2 (SEQ ID No:7) and P8 (SEQ ID No: 13) were used for PCR. Primer P8 has random nucleotides, which are depicted in SEQ ID NO: 13 by the letter "n" (for A or G or C or T). Conditions for PCR were as follows: denaturation step for 3 min at 95°C; profile for two first cycles: 1 min at 95°C, 30 sec at 50°C, 40 sec at 72°C; profile for the last 25 cycles: 20 sec at 94°C, 20 sec at 60°C, 15 sec at 72°C; final step: 5 min at 72°C. Both amplified DNA fragments were purified by agarose gel electrophoresis. Then, the obtained DNA fragments were treated with endonuclease *Bgl*II followed by ligation of the fragments in equimolar proportion. The mixture for ligation was incubated at 4°C overnight and was then used as a template for the next PCR procedure, and primers P1 (SEQ ID No:6) and P2 (SEQ ID No:7) were used for the PCR. Conditions for PCR were as follows: denaturation step for 3 min at 95°C; profile for two first cycles: 1 min at 95°C, 30 sec at 50°C, 40 sec at 72°C; profile for the last 12 cycles: 20 sec at 94°C, 20 sec at 60°C, 15 sec at 72°C; final step: 5 min at 72°C.

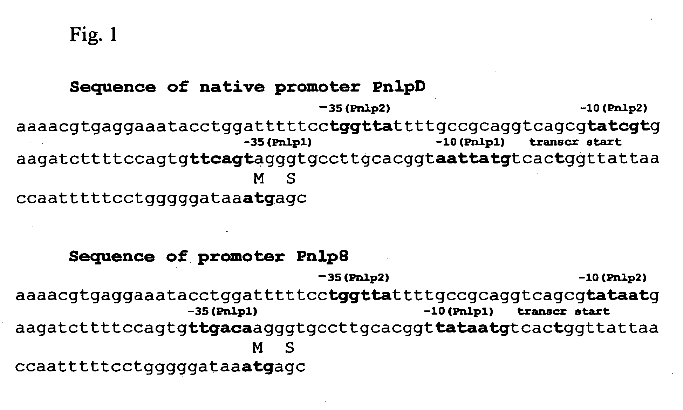

The amplified DNA fragment was about 0,2 kb in size, and was purified by agarose gel electrophoresis. Then, the purified fragment was treated with endonucleases *Pae*I and *Sal*I*.* The obtained DNA fragment was then ligated with plasmid pMIV-Pnlp0-yeaS3 which had been previously treated with endonucleases *Pae*I and *Sal*I*.* The mixture for ligation was incubated at 4°C overnight and was then used to transform *E. coli* MG1655 strain by electroporation. The resulting transformants were plated on plates with LB agar containing ampicillin (50mg/l), and the plates were incubated at 37°C overnight until individual colonies were visible. Plasmids were isolated from the obtained transformants and analyzed by sequencing analysis. The obtained plasmid containing promoter P*ₙₗₚ₈* (Fig.1) was named pMIV-Pnlp8-yeaS7.

Then, the plasmid pMIV-Pnlp8-yeaS7 was treated with endonuclease *Hind*III followed by purification and treatment with DNA polymerase I large fragment (Klenow fragment). The obtained DNA fragment was purified and treated with endonuclease *Nco*I*.* The obtained DNA fragment after purification was then ligated in equimolar proportion into plasmid pMW-Pomp-cysE5 (WO2005007841) which had been previously treated with endonucleases *Sma*I and *Nco*I*.* The mixture for ligation was incubated at 4°C overnight and was then used to transform *E. coli* MG1655 strain by electroporation. The resulting transformants were plated on plates with LB agar containing ampicillin (50mg/l), and the plates were incubated at 37°C overnight until individual colonies were visible. Plasmids were isolated from the obtained transformants and analyzed by restriction analysis. The obtained plasmid containing *cysE5* was named pMIV-EY2. Enzymatic activity of serine acetyltransferase was measured in the obtained transformant in order to confirm the intactness of the *cysE5* allele.

The next step was to integrate the *cysE5* and *yeaS* genes into the chromosome of *P. ananatis* SC17(US patent 6,596,517) strain. Plasmid pMH10 (Zimenkov D. et al., Biotechnologiya (in Russian), 6, 1-22 (2004)) was used to transform *P. ananatis* strain SC 17 by electroporation. The resulting transformants were plated on plates with LB agar containing kanamycin (20mg/l), the plates were incubated at 30°C overnight until individual colonies became visible. The obtained strain SC17/pMH10 was reseeded two times. Then,the plasmid pMIV-EY2 was used to transform the *P. ananatis* strain SC17/pMH10 (this strain was grown at 30°C) by electroporation. The resulting transformants were shocked by incubation at high temperature (42°C, 20 min) and plated on plates with LB agar containing chloramphenicol (20mg/l), the plates were incubated at 39°C overnight until individual colonies were visible. About 50 clones were reseeded at 39°C, and each of them were inoculated in 1 ml of LB medium and incubated at 39°C for 48 hours. After incubation, all 50 variants were tested for curing from the plasmids pMH10 and pMIV-EY2, and variants were selected which were resistant to chloramphenicol (20mg/l) but sensitive to kanamycin (20mg/l) and ampicillin (50mg/l). The desired integrants were identified by PCR analysis using primers P1 and P6. The obtained line of strains was named as EY01-EY50 and all of them were tested for the ability to produce cysteine in test-tubes fermentation. The best producer strain EY19 was selected and used in the following experiments.

To cure the *P. ananatis* strain EY19 of resistance to chloramphenicol, strain EY19 was transformed with the plasmid pMT-Int-Xis2 (WO 2005 010175) using electroporation. The resulting transformants were plated on plates with LB agar containing tetracycline (10mg/l), and the plates were incubated at 30°C overnight until individual colonies were visible. The desired transformants were identified by selecting variants which were sensitive to chloramphenicol (20mg/l). The obtained "cured" strain was named EY19(s).

The next step was to substitute the promoter region of *cysPTWA* genes by P*ₙₗₚ₈* promoter region into the strain EY19(s). PCR was carried out using the plasmid pMIV-Pnlp8-yeaS7 as a template and primers P1 (SEQ ID No:6) and P2 (SEQ ID No:7). Conditions for PCR were as follows: denaturation step for 3 min at 95°C; profile for two first cycles: 1 min at 95°C, 30 sec at 50°C, 40 sec at 72°C; profile for the last 20 cycles: 20 sec at 94°C, 20 sec at 59°C, 15 sec at 72°C; final step: 5 min at 72°C. The amplified DNA fragment was about 0,2kb in size, and was purified by agarose gel electrophoresis. Then, the purified fragment was treated with Klenow fragment. The resulting DNA fragment was then ligated in equimolar proportion with the plasmid pMW1 18-(λattL-Km^{r}-λ*attR*) (see Reference example 2) which had been previously treated with endonuclease *Xba*I followed by treatment with Klenow fragment. The mixture for ligation was incubated at 4°C overnight and was then used to transform *E. coli* MG1655 strain by electroporation. The resulting transformants were plated on plates with LB agar containing kanamycin (20mg/l), and the plates were incubated at 37°C overnight until individual colonies were visible. Plasmids were isolated from the obtained transformants and analyzed by restriction analysis. The obtained plasmid containing P*ₙₗₚ₈* promoter was named pMW-Km-Pnlp8. Then, PCR was carried out using the plasmid pMW-Km-Pnlp8 as a template and primers P9 (SEQ ID No:14) and P10 (SEQ ID No:15). Conditions for PCR were as follows: denaturation step for 3 min at 95°C; profile for two first cycles: 1 min at 95°C, 30 sec at 50°C, 40 sec at 72°C; profile for the last 30 cycles: 20 sec at 94°C, 20 sec at 54°C, 90 sec at 72°C; final step: 5 min at 72°C. The obtained DNA fragment was about 1,6 kb in size, and was purified by agarose gel electrophoresis and used to transform *P. ananatis* SC 17 strain by electroporation. The resulting transformants were plated on plates with LB agar containing kanamycin (20mg/l), and the plates were incubated at 34°C overnight until individual colonies were visible. The desired transformants were identified by PCR analysis using primers P1 (SEQ ID No:16) and P12 (SEQ ID No:17). The obtained strain was named SC17-Pnlp8-PTWA. Chromosomal DNA was isolated from the strain SC17-Pnlp8-PTWA. 10µg of this chromosomal DNA was used to transform *P. ananatis* EY19(s) by electroporation. The resulting transformants were plated on plates with LB agar containing kanamycin (20mg/l), and the plates were incubated at 34°C overnight until individual colonies were visible. The desired transformants were identified by PCR analysis using primers P11 and P12. The obtained strain was named EYP197. To cure the *P. ananatis* strain EYP197 of resistance to kanamycin, strain EYP197 was transformed with the plasmid pMT-Int-Xis2 by electroporation. The resulting transformants were plated on plates with LB agar containing tetracycline (10 mg/l), and the plates were incubated at 30°C overnight until individual colonies were visible. The desired transformants were identified by selecting variants which were sensitive to kanamycin (20 mg/l). The obtained "cured" strain was named EY197(s).

Mutation N348A was introduced by site-specific mutagenesis. For this purpose, the 3'-end of the *serA* gene (with mutation) which encodes phosphoglycerate dehydrogenase was obtained by PCR amplification using chromosomal DNA of the strain SC 17 as a template and primers P13 (SEQ ID No:18) and P14 (SEQ ID No:19), and the 5'-end of *serA* gene was obtained by PCR amplification using from the chromosomal DNA of the strain SC 17 as a template and primers P15 (SEQ ID No:20) and P16 (SEQ ID No:21). Conditions for the first PCR were as follows: denaturation step for 3 min at 95°C; profile for two first cycles: 1 min at 95°C, 30 sec at 50°C, 40 sec at 72°C; profile for the last 25 cycles: 20 sec at 94°C, 20 sec at 60°C, 60 sec at 72°C; final step: 5 min at 72°C. Conditions for the second PCR were as follows: denaturation step for 3 min at 95°C; profile for two first cycles: 1 min at 95°C, 30 sec at 50°C, 40 sec at 72°C; profile for the last 20 cycles: 20 sec at 94°C, 20 sec at 60°C, 20 sec at 72°C; final step: 5 min at 72°C. Both amplified DNA fragments were purified by agarose gel electrophoresis followed by treatment with endonuclease *Sma*I*.* The obtained DNA fragments were then ligated in equimolar proportion. The mixture for ligation was incubated at 4°C overnight and was used as a template for the next PCR procedure (with primers P13 and P15 (SEQ ID No:20)). Conditions for PCR were as follows: denaturation step for 3 min at 95°C; profile for two first cycles: 1 min at 95°C, 30 sec at 50°C, 40 sec at 72°C; profile for the last 15 cycles: 20 sec at 94°C, 20 sec at 60°C, 75 sec at 72°C; final step: 5 min at 72°C. The amplified DNA fragment was about 1,3kb in size, and it was purified by agarose gel electrophoresis. The obtained fragment was treated with endonucleases *Sal*I and *Xba*I*.* After restriction, the DNA fragment was ligated in equimolar proportion with the plasmid pMIV-Pnlp8-ter which had been previously treated with endonucleases *Sal*I and *Xba*I*.* The mixture for ligation was incubated at 4°C overnight and was then used to transform *E. coli* MG1655 by electroporation. The resulting transformants were plated on plates with LB agar containing ampicillin (50mg/l), and the plates were incubated at 37°C overnight until individual colonies were visible. Plasmids were isolated from the obtained transformants and analyzed by sequencing analysis. The obtained plasmid, containing the *serA* gene with the mutation N348A, was named pMIV-Pnlp8-serA348.

The next step was to integrate the *serA348* allele into chromosome of the *P. ananatis* strain SC17. Plasmid DNA pMIV-Pnlp8-serA348 was used to transform *P. ananatis* strain SC17/pMH10 (this strain was grown at 30°C) by electroporation. The resulting transformants were shocked by incubation at a high temperature (42°C, 20 min) and plated on plates with LB agar containing chloramphenicol (20mg/l), and the plates were incubated at 39°C overnight until individual colonies were visible. About 50 clones were reseeded at 39°C and then each of them were inoculated in 1 ml of LB medium and incubated at 39°C for 48h. After incubation all 50 variants were tested for curing of the plasmids pMH10 and pMIV-Pnlp8-serA348 by selecting variants which were resistant to chloramphenicol (20mg/l) but sensitive to kanamycin (20mg/l) and ampicillin (50mg/l). The desired integrants were identified by PCR analysis using primers P1 and P15. In all the obtained variants, specific activity of PGD was measured and the most active one was selected for the following purpose. It was named SC7int-serA348.

The next step was to transfer an integrated copy of *serA348* into the strain EYP197(s):

Chromosome DNA was isolated from the strain SC17int-serA348. 10µg of this chromosomal DNA was used to transform *P. ananatis* EYP197(s) by electroporation. The resulting transformants were plated on plates with LB agar containing chloramphenicol (20mg/l), and the plates were incubated at 34°C overnight until individual colonies were visible. The desired transformants were identified by PCR analysis using primers P1 and P15. The obtained strain was named EYPS 1976.

The next step was to introduce the *gcd* deletion into the strain EYPS1976. To cure the *P. ananatis* strain EYPS1976 of resistance to the chloramphenicol strain, EYPS 1976 was transformed with the plasmid pMT-Int-Xis2 by electroporation. The resulting transformants were plated on plates with LB agar containing tetracycline (10mg/l), and the plates were incubated at 30°C overnight until individual colonies were visible. The desired transformants were identified by selecting variants which were sensitive to chloramphenicol (20mg/l). The obtained "cured" strain was named EYPS1976(s).

The strain *P. ananatis* SC17(0) in which the *gcd* gene is deleted was constructed by the method initially developed by Datsenko, K.A. and Wanner, B.L. (Proc. Natl. Acad. Sci. USA, 2000, 97(12), 6640-6645) called "Red-driven integration". The DNA fragment containing the Km^{R} was obtained by PCR using primers P17 (SEQ ID NO: 28) and P18 (SEQ ID NO: 29) and plasmid pMW118-attL-Km-attR-ter_rrnB (Reference example 2) as a template. The obtained PCR product was purified in agarose gel and was used for electroporation of the strain *P. ananatis* SC 17(0), which contains the plasmid pKD46 having a temperature-sensitive replication. The plasmid pKD46 (Datsenko, K.A. and Wanner, B.L., Proc. Natl. Acad. Sci. USA, 2000, 97:12:6640-45) includes a 2,154 nucleotide DNA fragment of phage λ (nucleotide positions 31088 to 33241, GenBank accession no. J02459), and contains genes of the λ Red homologous recombination system (γ, β, exo genes) which are under the control of the arabinose-inducible P_{araB} promoter. The plasmid pKD46 is necessary for integration of the PCR product into the chromosome of strain SC17(0). The mutants having the *gcd* gene deleted and marked with the Km resistance gene were verified by PCR. Locus-specific primers P37 (SEQ ID NO: 54) and P38 (SEQ ID NO: 28) were used in PCR for the verification. The PCR product obtained in the reaction with the cells of parental gcd ⁺ strain SC 17(0) as a template, was 2560 bp in llength. The PCR product obtained in the reaction with the cells of mutant strain as a template was 1541 bp in length. The mutant strain was named SC17(0)Δgcd::Km.

Chromosomal DNA was isolated from the strain SC17Δgcd::Km. 10µg of this chromosomal DNA was used to transform *P. ananatis* EYPS 1976(s) by electroporation. The resulting transformants were plated on plates with LB agar containing kanamycin (20mg/l), and the plates were incubated at 34°C overnight until individual colonies became visible. The desired transformants were identified by PCR analysis using primers P17 (SEQ ID No:28) and P18 (SEQ ID No:29). The obtained strain was named EYPSG8.

To cure the *P. ananatis* strain EYPSG8 of resistance to kanamycin, strain EYPSG8 was transformed with the plasmid pMT-Int-Xis2 by electroporation. Theh resulting transformants were plated on plates with LB agar containing tetracycline (10mg/l), and the plates were incubated at 30°C overnight until individual colonies were visible. The desired transformants were identified by selecting variants which were sensitive to kanamycin (20mg/l). The obtained "cured" strain was named EYPSG8(s).

### 2.Construction of the strain EYPSG-Pnlp8-cysGDNC

The promoter region of the *cysGDNC* genes in the strain EYPSG8(s) was substituted with the P*ₙₗₚ₈* promoter. PCR was carried out using the plasmid DNA pMW-Km-Pnlp8 as a template and primers P19 (SEQ ID No:24) and P20 (SEQ ID No:25). Conditions for PCR were as follows: denaturation step for 3 min at 95°C; profile for two first cycles: 1 min at 95°C, 30 sec at 50°C, 40 sec at 72°C; profile for the last 30 cycles: 20 sec at 94°C, 20 sec at 54°C, 90 sec at 72°C; final step: 5 min at 72°C. The amplified DNA fragment was about 1,6 kb in size and was purified by agarose gel electrophoresis. The obtained fragment was used to transform *P. ananatis* SC 17 strain by electroporation. The resulting transformants were plated on plates with LB agar containing kanamycin (20mg/l), and the plates were incubated at 34°C overnight until individual colonies were visible. The desired transformants were identified by PCR analysis using primers P21 (SEQ ID No:26) and P22 (SEQ ID No:27). The obtained strain was named SC17-Pnlp8-cysGDNC. To transfer *cysGDNC* under control of the promoter P*ₙₗₚ₈* into the strain EYPSG8(s), chromosomal DNA was isolated from the strain SC17-Pnlp8-cysGDNC. 10µg of this chromosomal DNA was used to transform *P. ananatis* EYPSG8(s) by electroporation. The resulting transformants were plated on plates with LB agar containing kanamycin (20mg/l), and the plates were incubated at 34°C overnight until individual colonies were visible. The desired transformants were identified by PCR analysis using primers P21 and P22. The obtained strain was named EYPSG-Pnlp8-cysGDNC.

To cure the *P. ananatis* strain EYPSG-Pnlp8-cysGDNC from resistance to kanamycin, strain EYPSG-Pnlp8-cysGDNC was transformed with the plasmid pMT-Int-Xis2 by electroporation. The resulting transformants were plated on plates with LB agar containing tetracycline (10mg/l), and the plates were incubated at 30°C overnight until individual colonies were visible. The desired transformants were identified by selecting variants which were sensitive to kanamycin (20mg/l). The obtained "cured" strain was named EYPSG-Pnlp8-cysGDNC(s).

### Example 2.Construction of a strain with enhanced expression of both the genes of cysGDNC cluster and the cysQ gene

First, the promoter region of the *cysQ* gene in the strain EYPSG-Pnlp8-cysGDNC(s) was substituted with the P*_{cysK}* promoter region. PCR was carried out using the plasmid DNA pMW-Km-PcysK as a template and primers P10 (SEQ ID No: 15) and P24 (SEQ ID No:29). The plasmid pMW-Km-PcysK was obtained by inserting a DNA fragment containing the promoter region of the *cysK* gene from *P. ananatis,* which was obtained by PCR using chromosomal DNA of the strain SC 17 as a template and primers P25 (SEQ ID No:30) and P26 (SEQ ID No:31), into the plasmid pMW1 18-attL-Km-attR-ter_rrnB, prior to ligation and sequentially treated with restrictase XbaI and Klenow fragment of DNA-polymerase I. Conditions for PCR were as follows: denaturation step for 3 min at 95°C; profile for two first cycles: 1 min at 95°C, 30 sec at 50°C, 40 sec at 72°C; profile for the last 30 cycles: 20 sec at 94°C, 20 sec at 54°C, 90 sec at 72°C; final step: 5 min at 72°C. The amplified DNA fragment was about 1,6 kb in size, and it was purified by agarose gel electrophoresis. The obtained fragment was used to transform *P. ananatis* SC17 strain by electroporation. The resulting transformants were plated on plates with LB agar containing kanamycin (20mg/l), and the plates were incubated at 34°C overnight until individual colonies were visible. The desired transformants were identified by PCR analysis using primers P25and P26. The obtained strain was named SC17-PcysK-*cysQ.*

Then, *cysQ* under the control of the promoter P*_{cysK}* was transferred into the strain EYPSG-Pnlp8-cysGDNC(s). Chromosomal DNA was isolated from the strain SC17-PcysK-cysQ. 10 µg of this chromosomal DNA was used to transform *P. ananatis* EYPSG-Pnlp8-cysGDNC(s) by electroporation. The resulting transformants were plated on plates with LB agar containing kanamycin (20mg/l), and the plates were incubated at 34°C overnight until individual colonies were visible. The desired transformants were identified by PCR analysis using primers P21 and P22. The obtained strain was named EYPSG-Pnlp8-cysGDNC-PcysK-cysQ.

### Example 3. Construction of a strain with enhanced expression of the cysQ gene

The gene *cysQ* under the control of the promoter P*_{cysK}* was transferred into the strain EYPSG8(s). The chromosomal DNA was isolated from the strain SC17-PcysK-cysQ. 10 µg of this chromosomal DNA was used to transform *P. ananatis* EYPSG8(s) by electroporation. The resulting transformants were plated on plates with LB agar containing kanamycin (20 mg/l), and the plates were incubated at 34°C overnight until individual colonies were visible. The desired transformants were identified by PCR analysis using primers P25 and P26. The obtained strain was named EYPSG- PcysK-cysQ.

### Example 4. Production of L-cysteine by P. ananatis strains

To test the effect of the enhanced expression of the genes involved in the process of sulphur assimilation on L-cysteine production, the productivities of the strains *P. ananatis* EYPSG8, EYPSG8-Pnlp-cysGDNC, EYPSG8-Pnlp-cysGDNC-PcysK-cysQ and EYPSG8-PcysK-cysQ were compared.

The strains *P. ananatis* EYPSG8, EYPSG8-Pnlp-cysGDNC, EYPSG8-Pnlp-cysGDNC-PcysK-cysQ and EYPSG8-PcysK-cysQ were grown for 17 hours at 34°C on L-agar plates. Then cells from about 30 cm² of the plate surface were inoculated into a 500-ml flask with L- medium (50ml) and cultivated with aeration for 5 hours at 32°C. After that, cultures were transferred into 450ml of the fermentation medium in the Jar-fermentors (Marubishi).

After cultivation, the amount of L-cysteine which had accumulated in the medium was determined by the method described by Gaitonde M.K. (Biochem J.;104(2):627-33(1967)) with some modifications as follows: 150µl of sample was mixed with 150µl of 1M H₂SO₄, incubated for 5 min at 20°C, then 700 µl H₂O was added to the mixture, 150 µl of the obtained mixture was transferred into the new vial and 800 µl of solution A(1M Tris pH8.0, 5mM DTT) was added. The obtained mixture was incubated for 5 min at 20°C, centrifugated for 10 min at 13000 rpm, and then 100 µl of the mixture was transferred into a 20x200-mm test tube. Then, 400 µl H₂O, 500 µl ice acetic acid, and 500 µl of solution B (0,63g ninhydrin; 10ml, ice acetic acid; 10ml, 36% HCl) were added, and the mixture was incubated for 10 min in a boiling water bath. Then 4.5 ml ethanol was added and the OD₅₆₀ was determined. The concentration of cysteine was calculated using the formula C(cys g/l)=11.3* OD₅₆₀. The results of 3 independent jar- fermentations are shown in Table 1. As it can be seen from the Table 1, EYPSG8-Pnlp8-cysGDNC, EYPSG8-Pnlp8-cysQ and EYPSG8-Pnlp8-cysGDNC-PcysK-cysQ caused a higher amount of accumulation of L-cysteine as compared with EYPSG8.

The composition of the fermentation medium (g/l) is as follows:

| | Concentration | Unit |
|---|---|---|
| (A) Glucose | 50 | g/L |
| MgSO₄ 7H₂O | 0.3 | g/L |
| (B)Tryptone Difco | 2 | g/L |
| Yeast extract Difco | 1 | g/L |
| (NH4)₂SO₄ | 15 | g/L |
| KH₂PO₄ | 1.5 | g/L |
| NaCl | 0.5 | g/L |
| L-histidine HCl | 0-0.1 | g/L |
| L-methionine | 0-0.35 | g/l |
| FeSO₄ 7H₂O | 2 | mg/l |
| CaCl₂ | 15 | mg/l |
| Na citrate SH₂O | 1 | g/l |
| Na₂MoO₄ H₂O | 0.15 | mg/l |
| CoCl₂ 6H₂O | 0.7 | mg/l |
| MnCl₂ 4 H₂O | 1.6 | mg/l |
| ZnSO₄ 7 H₂O | 0.3 | mg/l |
| GD 113 | 0.03 | ml/l |
| (C) Pyridoxine (Na-salt) | 2 | mg/l |

**Table 1**

| Strain | Amount of L-cysteine, g/l |
|---|---|
| EYPSG8-Pnlp8 | 0.85 ± 0.07 |
| EYPSG8-Pnlp8-cysGDNC | 1.37 ± 0.09 |
| EYPSG8-PcysK-cysQ | 1.06 ± 0.08 |
| EYPSG8-Pnlp8-cysGDNC-PcysK-cysQ | 1.69 ± 0.11 |

### Example 5. Production of L-cysteine by E. coli strains

To test the effect of enhanced expression of the *cysQ* gene from *E. coli* on L-cysteine production, *E. coli* strain MT/pACYC-DES/pMIV-PompC-cysQ was constructed. For that purpose, *E. coli* strain MT/pACYC-DES (European patent EP1528108B1) was transformed with plasmid pMIV-PompC-cysQ. Plasmid pMIV-PompC-cysQ was constructed as follows.

At first, the promoter region of the *ompC* gene from *E. coli* was amplified by PCR from the chromosomal DNA of the *E*. *coli* strain MG1655 using the primers PrOMPCF (SEQ ID NO: 55) and PrOMPCR (SEQ ID NO: 56). The 0,3kb fragment was isolated, digested with *PaeI* and *Sal*I restrictases and ligated into the pMIV-5JS plasmid which had been previously treated with the same restrictases.

Then, the *cysQ* gene was amplified by PCR from the chromosome of the strain MG1655 using the primers mz017 (SEQ ID NO: 57) and mz018 (SEQ ID NO: 58). The 0,76kb PCR fragment was isolated, digested by *Sal*I and *Xba*I restrictases, and was cloned into SalI/XbaI sites of the plasmid obtained earlier. The resulting plasmid was named pMIV-PompC-cysQ.

The strain MT was constructed from the strain MG1655 as follows. Initially, a mutation in the *metC* gene was induced by N-methyl-N'-nitro-N-nitrosoguanidine (NTG) treatment followed by multiple procedures of ampicillin enrichment. The mutant, which was able to grow on cystathionine, but not on homocysteine, was selected. A *metC-*dificient strain can also be obatined by recombinant DNA techniques according to the method described in United State Patent No. 6,946,268.

Then, the disrupted *tnaA* gene from the strain CGSC7152 (tnaA300::Tn10(TcR), *E. coli* Genetic Stock Center, USA) was transferred into the resulting metC⁻ strain by the standard procedure of P1 transduction (Sambrook et al, "Molecular Cloning A Laboratory Manual, Second Edition", Cold Spring Harbor Laboratory Press (1989)). The plasmid pACYC-DES was constructed by inserting *ydeD* gene, mutant *cysE* gene and mutant *serA5* gene into the vector pACYC 184 under P_{ompA} promoter. The *ydeD* gene, which encodes a membrane protein not involved in the biosynthetic pathway of any L-amino acid, can enhance production of L-cysteine when additional copies of the gene are introduced into cells of the respective producing strain (US patent 5,972,663). The *serA5* gene encodes feed-back resistant phosphoglycerate dehydrogenase (US patent 6,180,373).

Strains MT/pACYC-DES and MT/pACYC-DES/pMIV-PompC-cysQ were cultivated overnight with shaking at 34 °C in a 2 ml of nutrient broth supplemented with 100 mg/l of ampicillin and 25 µg/ml of streptomycin. 0.2 ml of the obtained cultures were inoculated into 2 ml of a fermentation medium containing tetracycline (20 mg/l) and ampicillin (100 mg/l) in 20 x 200 mm test tubes, and cultivated at 34 °C for 42 hours with a rotary shaker at 250 rpm. The composition of the fermentation medium was following: 15.0 g/l of (NH₄)₂SO₄, 1.5 g/l of KH₂PO₄, 1.0 g/l of MgSO₄, 20.0 g/l of CaCO₃, 0.1 mg/l of thiamine, 1% of LB, 4% of glucose, 300 mg/l of L-methionine.

After the cultivation, the amount of L-cysteine accumulated in the medium was determined by the method described by Gaitonde, M.K. (Biochem. J., 104:2, 627-33 (1967)). The obtained data are presented in the Table 2. As it can be seen from the Table 2, strain MT/pACYC-DES/pMIV-PompC-cysQ caused a higher amount of L-cysteine accumulation as compared with MT/pACYC-DES.

To test the effect of enhanced expression of the *cysDNC* operon, *cysDN* operon or *cysC* gene, and the effect of enhanced expression of both the *cysDNC* operon and the *cysQ* gene from *E. coli* on L-cysteine production, *E. coli* strains MT-intPcysK-cysDNC/pACYC-DES and MT-intPcysK-cysDNC/pACYC-DES/pMIV-PompC-cysQ were constructed as follows.

At first, the promoter region of the *cysK* gene from *E. coli* was amplified by PCR from the chromosomal DNA of the *E. coli* strain MG1655 using the primers N1 (SEQ ID NO: 59) and and N2 (SEQ ID NO: 60). The obtained fragment with P*_{cysK}* was isolated, digested with *Pae*I and *Sal*I restrictases and ligated into the pMIV-5JS plasmid which had been previously treated with the same restrictases. The resulting plasmid was named as pMIV-P*_{cysk}*.

Then, the *cysDNC* operon was cloned by PCR using the chromosome DNA of the strain MG1655 as a template and primers N3 (SEQ ID NO: 61) and N4 (SEQ ID NO: 62). The obtained PCR fragment with *cysDNC* operon was isolated, digested with *Sal*I and *XbaI* restrictases and ligated into the pMIV-P*_{cysK}* plasmid which had been previously treated with the same restrictases. The resulting plasmid was named as pMIV-P*_{cysK}*-cysDNC.

Further, the *cysDN* operon was cloned by PCR using the chromosome DNA of the strain MG1655 as a template and primers N3 (SEQ ID NO: 61) and N5 (SEQ ID NO: 63). The obtained PCR fragment with *cysDN* operon was isolated, digested with *SalI* and *XbaI* restrictases and ligated into the pMIV-P*_{cysK}* plasmid which had been previously treated with the same restrictases. The resulting plasmid was named as pMIV-P*_{cysK}*-cysDN.

Also, the *cysC* gene was cloned by PCR using the chromosome DNA of the strain MG1655 as a template and primers N6 (SEQ ID NO: 64) and N4 (SEQ ID NO: 62). The obtained PCR fragment with *cysDNC* operon was isolated, digested with *SalI* and *XbaI* restrictases and ligated into the pMIV-P*_{cysK}* plasmid which had been previously treated with the same restrictases. The resulting plasmid was named as pMIV-P*_{cysK}*-cysC.

Plasmids pMIV-P*_{cysK}* cysDNC, pMIV-P*_{cysK}* cysDN and pMIV-P*_{cysK}* cysC were used to transform the strain MT/pACYC-DES by electroporation to obtain strains MT/pACYC-DES/pMIV-PcysK-cysDNC, MT/pACYC-DES/pMIV-PcysK-cysDN and MT/pACYC-DES/pMIV-PcysK-cysC, respectively.

The next step was to integrate P*_{cysK}-*cysDNC fragment into chromosome of the strain *E. coli* MT. For this purpose; integration of *cysDNC* operon under control of P*_{cysK}* promoter was obtained as result mini-µu integration (with help of µu-transposase carrying plasmid pMH10) of the plasmid pMIV-P*_{cysK}*-cysDNC in the chromosome of the strain MG1655. Obtained construction (intP*_{cysK}* -cysDNC) was transferred from strain MG1655-int-P*_{cysK}*-cysDNC into the strain MT by P1 transduction (Miller, J.H. (1972) Experiments in Molecular Genetics, Cold Spring Harbor Lab. Press, Plainview, NY) to obtain *E. coli* strain MT-int-P*_{cysK}*-cysDNC. Plasmid pACYC-DES was used to transform the strain MT-int-P*_{cysK}*-cysDNC by electroporation to obtain *E. coli* strain MT-int-P*_{cysK}*-cysDNC/pACYC-DES, and both plasmids pACYC-DES and pMIV-PompC-cysQ were used to transform the strain MT-int-P*_{cysK}*-cysDNC by electroporation to obtain strain MT-int-P*_{cysK}*-cysDNC/pACYC-DES/pMIV-PompC-cysQ.

Strains MT-intPcysK-cysDNC/pACYC-DES and MT-intPcysK-cysDNC/pACYC-DES/pMIV-PompC-cysQ containing *cysDNC* operon integrated into the chromosome, and strains MT/pACYC-DES/C, MT/pACYC-DES/pMIV-PcysK-cysDN and MT/pACYC-DES/pMIV-PcysK-cysC containing *cysDNC* operon, *cysDN* operon and *cysC* gene on the pMIV plasmid, were separately cultivated overnight with shaking at 34 °C in a 2 ml of nutrient broth supplemented with 100 mg/l of ampicillin and 20 µg/ml of tetracycline. 0.2 ml of the obtained cultures were inoculated into 2 ml of a fermentation medium containing tetracycline (20 mg/l) and ampicillin (100 mg/l) in 20 x 200 mm test tubes, and cultivated at 34 °C for 42 hours with a rotary shaker at 250 rpm. The composition of the fermentation medium was following: 15.0 g/l of (NH₄)₂SO₄, 1.5 g/l of KH₂PO₄, 1.0 g/l of MgSO₄, 20.0 g/l of CaCO₃, 0.1 mg/l of thiamine, 1% of LB, 4% of glucose, 300 mg/l of L-methionine.

After the cultivation, the amount of L-cysteine accumulated in the medium was determined by the method described by Gaitonde, M.K. (Biochem. J., 104:2,627-33 (1967)). The obtained data are presented in the Table 2. As it can be seen from the Table 2, strain MT-intPcysK-cysDNC/pACYC-DES had slight positive effect on L-cysteine production as compared with control strain MT/pACYC-DES, strain MT-intPcysK-cysDNC/pACYC-DES/pMIV-PompC-cysQ caused a significantly higher amount of L-cysteine accumulation as compared with MT/pACYC-DES. Also strain MT/pACYC-DES/pMIV-PcysK-cysC had slight positive effect on L-cysteine production, strains MT/pACYC-DES/pMIV-PcysK-cysDNC and MT/pACYC-DES/pMIV-PcysK-cysDN cuased a significantly higher amount of L-cysteine accumulation as compared with MT/pACYC-DES. Highest amount of L-cysteine accumulation was observed when *cysDNC* operon uner control of P*_{cysK}* promoter was combined with *cysQ* gene under control of P*_{ompC}* promoter in one strain MT-intPcysK-cysDNC /pACYC-DES/pMIV-PompC-cysQ.

**Table 2**

| Strain | Amount of L-cysteine, g/l |
|---|---|
| MT/pACYC-DES | 1.05 ± 0.13 |
| MT/pACYC-DES/pMIV-PompC-cysQ | 1.46 ± 0.20 |
| MT-intPcysK-cysDNC /pACYC-DES | 0.85 ± 0.14 |
| MT-intPcysK-cysDNC /pACYC-DES/pMIV-PompC-cysQ | 0.85 ± 0.11 |
| MT/pACYC-DES/pMIV-PcysK-cysDNC | 1.67 ± 0.12 |
| MT/pACYC-DES/pMIV-PcysK-cysDN | 1.51 ± 0.11 |
| MT/pACYC-DES/pMIV-PcysK-cysC | 1.10 ± 0.10 |

### Example 6. Production of L-methionine by E. coli strains.

To test the effect of enhanced expression of the *cysDNC* operon, *cysDN* operon, *cysC* gene and *cysQ* gene from *E. coli* or combinations thereof on L-methionine production, *E. coli* strain 73 (VKPM B-8126) containing said operons or genes in different combinations integrated into the chromosome or/and cloned on plasmids pMIV-P*_{ompC}*-cysQ, pMIV-P*_{cysK}*-cysDNC, pMIV-P*_{cysK}*-cysDN, pMIV-P*_{cysK}*-cysC can be constructed as described above (Example 5). *E. coli* strain 73 (VKPM B-8126) has been deposited in the Russian National Collection of Industrial Microorganisms (VKPM) (Russia 113545 Moscow 1 Dorozhny proezd, 1) on May 14, 2001 under accession number VKPM B-8126, and transferred from the original deposit to international deposit based on Budapest Treaty on February 1, 2002.

*E. coli* strain 73 and obtained strains can be separately cultivated in 2 ml of minimal medium ((NH₄)₂SO₄ - 18 g/l, K₂HPO₄ - 1.8 g/l, MgSO₄ - 1.2 g/l, thiamin - 0.1 mg/l, yeast extract - 10 g/l, glucose - 60 g/l, threonine - 400 mg/l, ampicillin - 100 mg/l, if necessary) in 20-ml test tubes and can be incubated overnight with aeration at 32 °C. The 0.2 ml of each night culture can be transferred to the three 20-ml test tubes with 2 ml of fresh medium for fermentation with or without IPTG and cultivated at 32 °C for 48 hours with rotary shaker.

**Fermentation medium composition:**

| | |
|---|---|
| (NH₄)₂SO₄ | 18.0 g/l, |
| K₂HPO₄ | 1.8 g/l, |
| MgSO₄ | 1.2 g/l, |
| CaCO₃ | 20.0 g/l, |
| Thiamin | 0.1 mg/l, |
| Glucose | 60.0 g/l, |
| Threonine | 400 mg/l, |
| Yeast extract | 1.0 g/l, |
| Ampicillin | 100 mg/l, if necessary. |

After cultivation the plasmid stability and optical absorbance of the medium at 540 nm can be determined by conventional methods. Accumulated amount of methionine in the medium can be determined by TLC. Liquid phase composition for TLC is as follows: isopropanol - 80 ml, ethylacetate - 80 ml, NH₄OH (30 %) - 15 ml, H₂O - 45 ml.

### Reference example 1. Construction of the plasmid pMIV5JS

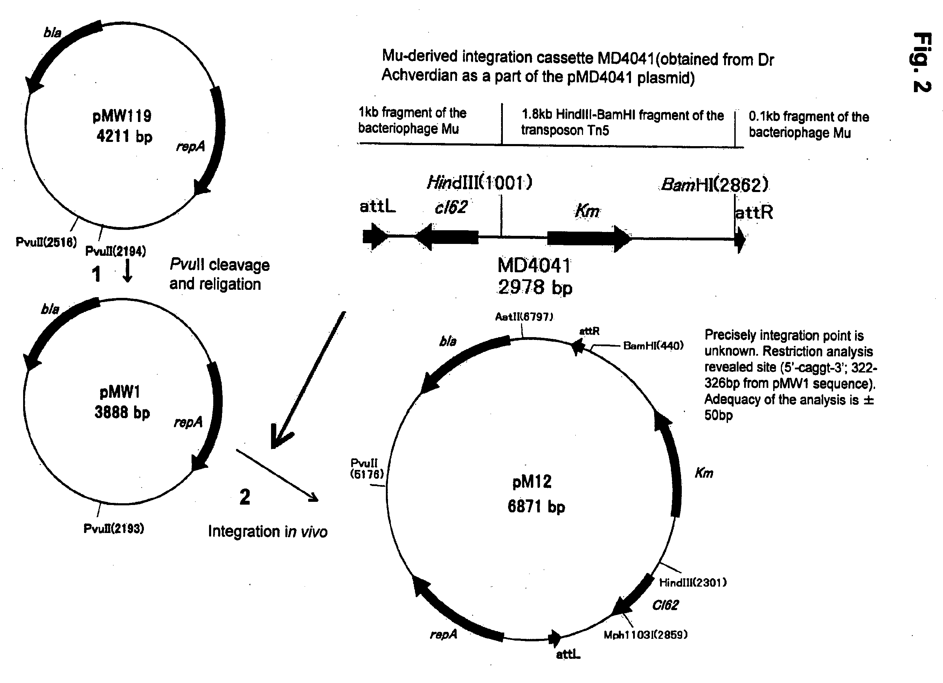

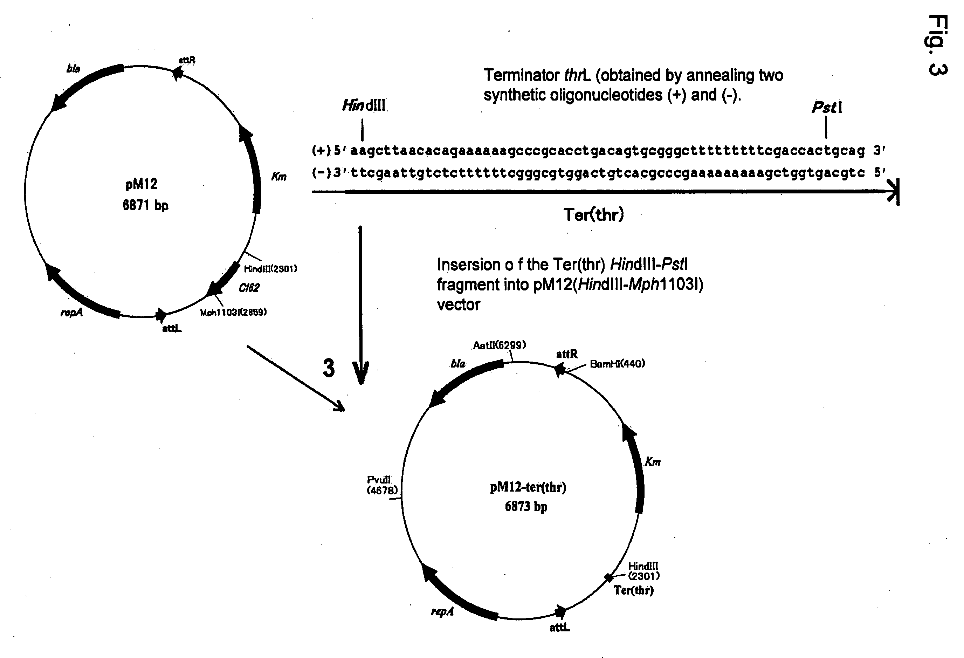

PMIV-5JS was constructed according to the following scheme. At first, plasmid pM12 was constructed by integrating *in vivo* a Mu-derived integration cassette into plasmid pMW1, which is derivative of pMW119 (Fig. 2). Two terminator oligonucleotide sequences complementary to each other were synthesized (SEQ ID NO: 32 and SEQ ID NO: 33). Terminator *thr*L was obtained by annealing these synthetic oligonucleotides in the forward (SEQ ID NO: 32) and reverse directions (SEQ ID NO: 33). Terminator *thr*L was flanked with sites *Hind*III and *Pst*I*.* Then plasmid pM12-ter(thr) was constructed by insertion of synthetic terminator sequence Ter(*thr*) into pM12 which had been digested with *Hind*III and *Mph*1103I (Fig. 3).

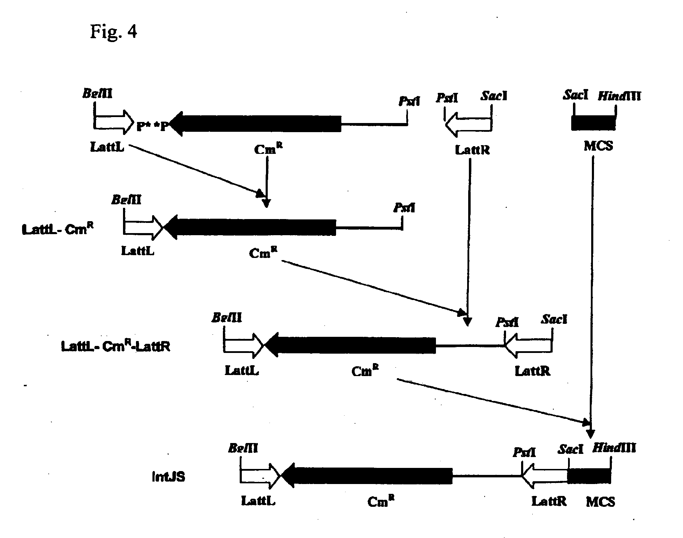

The intJS integrative cassette was constructed as following (Fig. 4):
a) a 0.12 kbp LattL fragment was obtained by PCR amplification using an upstream primer (SEQ ID NO: 34) (the site for *Bgl*II is underlined), and a phosphorylated downstream primer (SEQ ID NO: 35). Plasmid pMW118-attL-tet-attR-ter_rrnB was used as a template (WO2005/010175);
b) a 1.03 kbp Cm^{R} fragment was obtained by PCR amplification using a phosphorylated upstream primer (SEQ ID NO: 36), and a downstream primer (SEQ ID NO: 37) (the site for *Pst*I is underlined). Plasmid pACYC184 was used as a template;
c) a 0.16 kbp LattR fragment was obtained by PCR amplification using an upstream primer (SEQ ID NO: 38) (the site for *Pst*I is underlined), and a downstream primer (SEQ ID NO: 39) (the site for *Sac*I is underlined). Plasmid pMW118-attL-tet-attR-ter_rrnB was used as a template;
d) fragments LattL and Cm^{R} were ligated and the resulting 1.15 kbp fragment LattL-Cm^{R} was purified;
e) fragments LattL-Cm^{R} and LattR were digested by *Pst*I*,* ligated, and the resulting 1.31 kbp LattL-Cm^{R}-LattR fragment was purified;
f) a 70 bp double stranded DNA fragment containing multiple cloning sites (MCS) was obtained by annealing two synthesized oligonucleotides: oligonucleotide having sequence depicted in SEQ ID NO: 40 and another oligonucleotide having sequence complementary to SEQ ID NO: 40;
g) fragments LattL-Cm^{R}-LattR and MCS were digested by *Sac*I*,* ligated, and the resulting 1.38 kbp cassette LattL-Cm^{R}-LattR-MCS was purified;

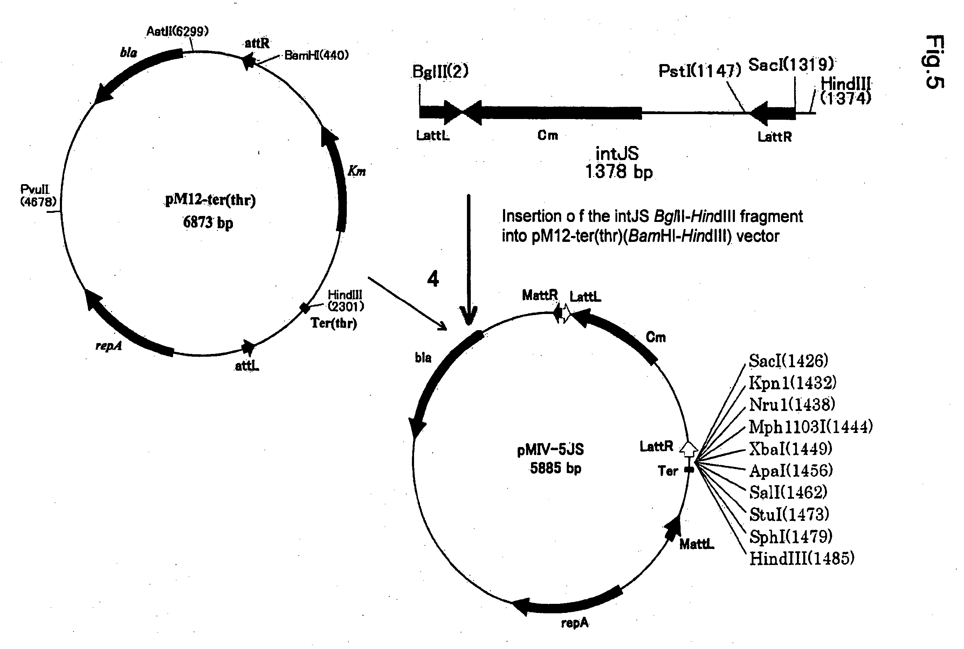

At the last step, the fragment LattL-Cm^{R}-LattR-MCS was digested by *Bgl*II and *Hind*III and cloned into pM12-ter(thr) which had been digested with *BamH*I and *Hind*III to yield plasmid pMIV-5JS (Fig. 5).

### Reference example 2. Construction of pMW118-(λattL-Km'-λattR) plasmid.

pMW118-(λattL-Km^{r}-λ*attR*) plasmid was constructed using the pMW118-attL-Tc-attR (WO2005/010175) plasmid and substituting the tetracycline resistance marker gene with the kanamycin resistance marker gene from pUC4K plasmid (Vieira, J. and Messing, J., Gene, 19(3): 259-68 (1982)).

For that purpose, the large *Eco*RI - *Hind*III fragment of pMW118-attL-Tc-attR plasmid was ligated to two fragments of pUC4K plasmid: *Hind*III - *Pst*I fragment (676 bp) and *Eco*RI - *Hind*III fragment (585 bp).

Basic pMW118-attL-Tc-attR was obtained by ligation of the following four DNA fragments:
1) the *Bgl*II*-EcoR*I fragment (114 bp) carrying *attL* (SEQ ID NO: 41) which was obtained by PCR amplification of the corresponding region of the *E. coli* W3350 (contained λ prophage) chromosome using oligonucleotides P27 and P28 (SEQ ID NOS: 42 and 43) as primers (these primers contained the subsidiary recognition sites for *Bgl*II and *EcoR*I endonucleases);
2) the *Pst*I*-Hind*III fragment (182 bp) carrying *attR* (SEQ ID NO: 44) which was obtained by PCR amplification of the corresponding region of the *E. coli* W3350 (contained λ prophage) chromosome using the oligonucleotides P29 and P30 (SEQ ID NOS: 45 and 46) as primers (these primers contained the subsidiary recognition sites for *Pst*I and *Hind*III endonucleases);
3) the large *Bgl*II*-Hind*III fragment (3916 bp) of pMW118-ter_*rrnB*.

The plasmid pMW118-ter_*rrnB* was obtained by ligation of the following three DNA fragments:
1) the large DNA fragment (2359 bp) carrying the *Aat*II*-EcoR*I fragment of pMW118 that was obtained in the following way: pMW118 was digested with *EcoR*I restriction endonuclease, treated with Klenow fragment of DNA polymerase I, and then digested with *Aat*II restriction endonuclease;
2) the small AatII-*Bgl*II fragment (1194 bp) of pUC 19 carrying the *bla* gene for ampicillin resistance (Ap^{R}) was obtained by PCR amplification of the corresponding region of the pUC19 plasmid using oligonucleotides P31 and P32 (SEQ ID NOS: 47 and 48) as primers (these primers contained the subsidiary recognition sites for *Aat*II and *Bgl*II endonucleases);
3) the small *Bgl*II*-Pst*I fragment (363 bp) of the transcription terminator ter_*rrnB* was obtained by PCR amplification of the corresponding region of the *E. coli* MG1655 chromosome using oligonucleotides P33 and P34 (SEQ ID NOS: 49 and 50) as primers (these primers contained the subsidiary recognition sites for *Bgl*II and *Pst*I endonucleases);
4) the small *EcoR*I*-Pst*I fragment (1388 bp) (SEQ ID NO: 51) of pML-Tc-ter_*thrL* bearing the tetracycline resistance gene and the ter_*thrL* transcription terminator; the pML-Tc-ter_*thrL* plasmid was obtained in two steps:
   - the pML-ter*_thrL* plasmid was obtained by digesting the pML-MCS plasmid (Mashko, S.V. et al., Biotekhnologiya (in Russian), 2001, no. 5, 3-20) with the *Xba*I and *BamH*I restriction endonucleases, followed by ligation of the large fragment (3342 bp) with the *Xba*I*-BamH*I fragment (68 bp) carrying terminator ter*_thrL* obtained by PCR amplification of the corresponding region of the *E. coli* MG1655 chromosome using oligonucleotides P35 and P36 (SEQ ID NOS: 52 and 53) as primers (these primers contained the subsidiary recognition sites for the *Xba*I and *BamH*I endonucleases);
   - the pML-Tc-ter_*thrL* plasmid was obtained by digesting the pML-ter_*thrL* plasmid with the *Kpn*I and *Xba*I restriction endonucleases followed by treatment with Klenow fragment of DNA polymerase I and ligation with the small *EcoR*I*-Van91*I fragment (1317 bp) of pBR322 bearing the tetracycline resistance gene (pBR322 was digested with *EcoR*I and *Van91*I restriction endonucleases and then treated with Klenow fragment of DNA polymerase I).

### Industrial Applicability

According to the present invention, production of L-cysteine, L-cystine, a derivative or precursor thereof or a mixture thereof by a bacterium of *Enterobacteriaceae* family can be improved.

## Claims

1. A method for producing a compound selected from the group consisting of L-cysteine, L-cystine, derivatives and precursors thereof, which comprises cultivating an L-cysteine producing bacterium of *Enterobacteriaceae* family in a culture medium containing sulphate, and collecting the compound from the culture medium,
wherein the bacterium has been modified to have enhanced expression of one or more genes selected from the group consisting of the genes of the *cysDNC* cluster and the *cysQ* gene,
wherein the derivatives are selected from the group consisting of S-sulfocysteine, thiazolidine derivatives, and hemithioketal, and the precursors are selected from the group consisting of O-acetylserine and N-acetylserine.

2. The method according to claim 1, wherein the bacterium has been modified to have enhanced expression of *cysQ* gene or *cysDN* genes, or both.

3. The method according to claim 2, wherein the bacterium has been further modified to have enhanced expression of *cysC* gene.

4. The method according to any of claims 1 to 3, wherein the expression of said gene(s) is/are enhanced by modifying an expression control sequence so that the expression of the gene(s) is/are enhanced.

5. The method according to claim 4, wherein native promoter(s) of said gene(s) is/are substituted with a more potent promoter(s)*.*

6. The method according to any one of claims 1 to 5, wherein the bacterium belongs to the genus *Pantoea.*

7. The method according to claim 6, wherein the bacterium is *Pantoea ananatis.*

8. The method according to any one of claims 1 to 5, wherein the bacterium belongs to the genus *Escherichia.*

9. The method according to claim 8, wherein the bacterium is *Escherichia coli.*

10. The method according to any one of claims 1 to 9, wherein the bacterium has enhanced expression of the genes involved in the biosynthesis of L-cysteine.

11. The method according to any one of claims 1 to 10, wherein the bacterium has enhanced expression of the genes involved in the biosynthesis of L-methionine.

## Patentansprüche

1. Verfahren zum Herstellen einer Verbindung, die aus der Gruppe ausgewählt ist, die aus L-Cystein, L-Cystin, Derivaten und Vorläufern davon besteht, welches das Kultivieren eines L-Cystein produzierenden Bakteriums der Familie Enterobacteriaceae in einem Sulfat enthaltenden Kulturmedium und das Gewinnen der Verbindung aus dem Kulturmedium umfasst, wobei das Bakterium so modifiziert worden ist, dass es eine erhöhte Expression eines oder mehrerer Gene aufweist, die aus der Gruppe ausgewählt sind, die aus den Genen des cysDNC-Clusters und dem cysQ-Gen besteht,
wobei die Derivate aus der Gruppe ausgewählt sind, die aus S-Sulfocystein, Thiazolidinderivaten und Hemithioketal besteht, und die Vorläufer aus der Gruppe ausgewählt sind, die aus 0-Acetylserin und N-Acetylserin besteht.

2. Verfahren nach Anspruch 1, wobei das Bakterium so modifiziert worden ist, dass es eine erhöhte Expression des cysQ-Gens und/oder der cysDN-Gene aufweist.

3. Verfahren nach Anspruch 2, wobei das Bakterium außerdem so modifiziert worden ist, dass es eine erhöhte Expression des cysC-Gens aufweist.

4. Verfahren nach einem der Ansprüche 1 bis 3, wobei die Expression des Gens oder der Gene durch Modifizieren einer Expressionskontrollsequenz erhöht ist, so dass die Expression des oder der Gene erhöht ist.

5. Verfahren nach Anspruch 4, wobei der native Promotor oder die nativen Promotoren des Gens oder der Gene durch einen oder mehrere stärkere Promotoren substituiert ist/sind.

6. Verfahren nach einem der Ansprüche 1 bis 5, wobei das Bakterium zu der Gattung Pantoea gehört.

7. Verfahren nach Anspruch 6, wobei das Bakterium Pantoea ananatis ist.

8. Verfahren nach einem der Ansprüche 1 bis 5, wobei das Bakterium zu der Gattung Escherichia gehört.

9. Verfahren nach Anspruch 8, wobei das Bakterium Escherichia coli ist.

10. Verfahren nach einem der Ansprüche 1 bis 9, wobei das Bakterium eine erhöhte Expression der an der Biosynthese von L-Cystein beteiligten Gene aufweist.

11. Verfahren nach einem der Ansprüche 1 bis 9, wobei das Bakterium eine erhöhte Expression der an der Biosynthese von L-Methionin beteiligten Gene aufweist.

## Revendications

1. Procédé de production d'un composé sélectionné dans le groupe consistant en la L-cystéine, la L-cystine, des dérivés et précurseurs de celles-ci, qui comprend la mise en culture d'une bactérie de la famille des *Enterobacteriaceae* produisant de la L-cystéine dans un milieu de culture contenant du sulfate, et la collecte du composé à partir du milieu de culture,
où la bactérie a été modifiée pour présenter une expression améliorée d'un ou de plusieurs gènes sélectionnés dans le groupe consistant en les gènes du cluster *cysDNC et* le gène *cysQ,*
où les dérivés sont sélectionnés dans le groupe consistant en la S-sulfocystéine, des dérivés de la thiazolidine et un hémithiocétal, et les précurseurs sont sélectionnés dans le groupe consistant en l'Oacétylsérine et la N-acétylsérine.

2. Procédé selon la revendication 1, dans lequel la bactérie a été modifiée pour présenter une expression améliorée du gène *cysQ* ou des gènes *cysDN,* ou les deux.

3. Procédé selon la revendication 2, dans lequel la bactérie a en outre été modifiée pour présenter une expression améliorée du gène *cysC.*

4. Procédé selon l'une quelconque des revendications 1 à 3, dans lequel l'expression dudit/desdits gène(s) est améliorée en modifiant une séquence de contrôle de l'expression de sorte que l'expression du/des gène(s) soit améliorée.

5. Procédé selon la revendication 4, dans lequel un/des promoteur(s) natif(s) dudit/desdits gène(s) est/sont substitué(s) par un/des promoteur(s) plus puissant(s).

6. Procédé selon l'une quelconque des revendications 1 à 5, dans lequel la bactérie appartient au genre *Pantoea.*

7. Procédé selon la revendication 6, dans lequel la bactérie est *Pantoea ananatis.*

8. Procédé selon l'une quelconque des revendications 1 à 5, dans lequel la bactérie appartient au genre *Escherichia.*

9. Procédé selon la revendication 8, dans lequel la bactérie est *Escherichia coli.*

10. Procédé selon l'une quelconque des revendications 1 à 9, dans lequel la bactérie présente une expression améliorée des gènes impliqués dans la biosynthèse de la L-cystéine.

11. Procédé selon l'une quelconque des revendications 1 à 10, dans lequel la bactérie présente une expression améliorée des gènes impliqués dans la biosynthèse de la L-méthionine.
